# EUROPEAN PATENT SPECIFICATION

(11) **EP 3 464 344 B1**
(45) Date of publication and mention of the grant of the patent: **13.12.2023**
(21) Application number: 17728602.8
(22) Date of filing: 31.05.2017
(51) Int. Cl.: C07K 14/725, C07K 16/28, C12N 5/078, C12N 5/0783, A61K 35/17, C07K 14/72, C07K 16/00, C07K 14/47, A61K 39/00, A61P 35/00

(54) **CELL EXPRESSING CAR AND GPCR**
ZELLE DIE CAR UND GPCR EXPRIMIERT
ZELLULE QUI EXPRIME CAR ET GPCR

(30) Priority: 01.06.2016 GB 201609604
(43) Date of publication of application: 10.04.2019
(73) Proprietor: UCL Business Ltd, London WC1E 6BT (GB)
(72) Inventor: PULÉ, Martin, London W1T 4TP (GB); MACIOCIA, Paul, London W1T 4TP (GB); KONG, Khai, London W1T 4TP (GB)
(74) Representative: D Young & Co LLP
(86) International application number: PCT/GB2017/051558
(87) International publication number: WO 2017/207992

(56) References cited:
- WO-A1-2016/055551
- WO-A2-2015/092024
- ROYBAL KOLE T ET AL: "Precision Tumor Recognition by T Cells With Combinatorial Antigen-Sensing Circuits", CELL, CELL PRESS, US, vol. 164, no. 4, 28 January 2016 (2016-01-28), pages 770-779, XP029416808, ISSN: 0092-8674, DOI: 10.1016/J.CELL.2016.01.011
- CHIA-YUNG WU ET AL: "Synthetic biology approaches to engineer T cells", CURRENT OPINION IN IMMUNOLOGY., vol. 35, 1 August 2015 (2015-08-01), pages 123-130, XP055422522, GB ISSN: 0952-7915, DOI: 10.1016/j.coi.2015.06.015

## Description

### FIELD OF THE INVENTION

The present invention relates to a cell comprising a chimeric antigen receptor (CAR).

### BACKGROUND TO THE INVENTION

Chimeric antigen receptor T-cells (CAR T-cells) graft the specificity of an antibody onto a T-cell. Patients with advanced B-cell malignancies have achieved deep and long-lasting remissions after CAR T-cell infusion, even in the setting of chemo-refractory disease. As such, there is growing interest in development of this therapeutic approach.

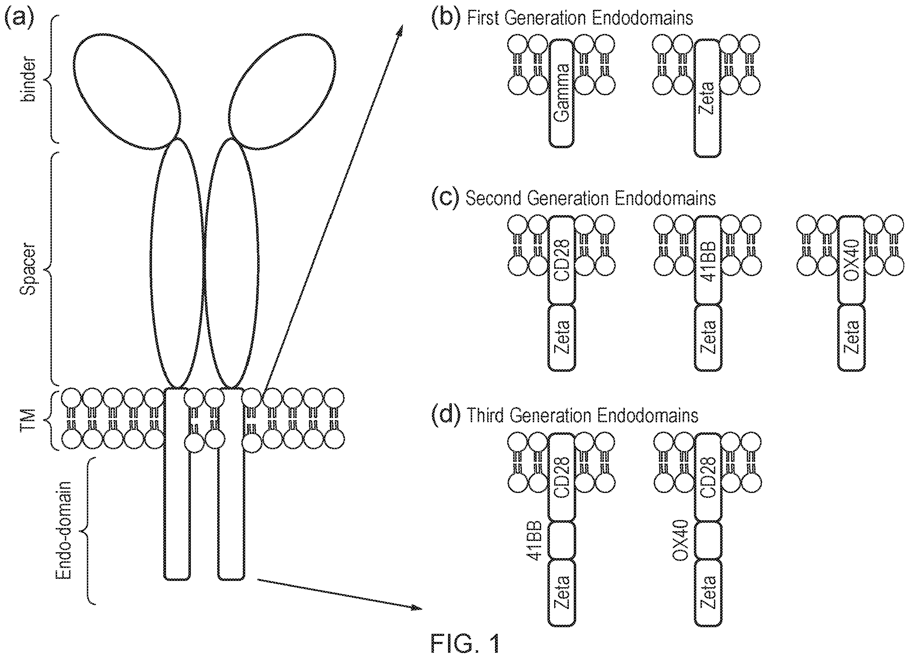

The usual structure of a CAR is that of a type I transmembrane domain protein with an antigen recognizing amino terminus, a spacer, a transmembrane domain all connected to a compound endodomain which transmits T-cell survival and activation signals (see Figure 1A).

The most common form of these molecules are fusions of single-chain variable fragments (scFv) derived from monoclonal antibodies which recognize a target antigen, fused via a spacer and a trans-membrane domain to a signaling endodomain. Such molecules result in activation of the T-cell in response to recognition by the scFv of its target. When T cells express such a CAR, they recognize and kill target cells that express the target antigen. Several CARs have been developed against tumour associated antigens, and adoptive transfer approaches using such CAR-expressing T cells are currently in clinical trial for the treatment of various cancers.

However, one important difficulty is the substantial overlap in marker expression on tumour cells and their non-mutated normal counterparts. This may lead to 'on-target off-tumour toxicity' with unwanted targeting of normal tissues.

For some cancers, targeting the presence of two cancer antigens may be more selective and therefore effective than targeting one. For example, B-chronic lymphocytic leukaemia (B-CLL) is a common leukaemia which is currently treated by targeting CD19. This treats the lymphoma but also depletes the entire B-cell compartment such that the treatment has a considerable toxic effect. B-CLL has an unusual phenotype in that CD5 and CD19 are co-expressed. By targeting only cells which express CD5 and CD19, it would be possible to considerably reduce off-target toxicity.

In other cancers, a tumour is best defined by presence of one antigen (typically a tissue-specific antigen) and the absence of another antigen which is present on normal cells. For example, acute myeloid leukaemia (AML) cells express CD33. Normal stem cells express CD33 but also express CD34, while AML cells are typically CD34 negative. Targeting CD33 alone to treat AML is associated with significant toxicity as it depletes normal stem cells. However, specifically targeting cells which are CD33 positive but not CD34 positive would avoid this considerable off-target toxicity.

Roybal et al. describe a combinatorially activated T cell circuit in which a synthetic Notch receptor for one antigen induces the expression of a CAR for a second antigen (Cell; 2016; 164; 770-779). Roybal *et al.* suggest that T cells expressing such a T cell circuit are only activated in the presence of dual antigen tumour cells.

Additional strategies which control CAR T-cells such that they kill only tumour, and not normal cells, would be desirable.

### SUMMARY OF ASPECTS OF THE INVENTION

The present inventors have developed CAR signalling systems in which the binding of a ligand to a G-protein coupled-receptor (GPCR) modulates CAR expression. Thus, CAR signalling in the present systems is dependent on the presence or absence of the GPCR ligand.

Although cancer is a genetically complex and heterogenous group of conditions, a pathological hallmark of malignant transformation is disordered metabolism. This disordered metabolism induces a tumour microenvironment of metabolites, for example, that differs from the microenvironment of normal cells and tissues.

The present invention couples GPCR recognition of a ligand - for example a metabolite which is enriched or depleted in a tumour microenvironment - to CAR expression and thereby enables CAR activity to be modulated.

Accordingly, in a first aspect the present invention provides a cell which co-expresses (i) a chimeric antigen receptor (CAR) and a G-protein coupled receptor (GPCR) at the cell surface and (ii) an intracellular component comprising a protease domain linked to a GPCR-targeting domain; wherein an intracellular domain of the GPCR comprises a protease cleavage site linked to a transcriptional regulatory domain, which transcriptional regulatory domain is capable of modulating the expression of a nucleic acid encoding the CAR; and wherein the protease domain of the intracellular component is capable of cleaving at the cleavage site; such that, upon binding of ligand to the GPCR, the intracellular component is recruited to the GPCR and the protease domain cleaves the cleavage site thereby releasing the transcriptional regulatory domain from the GPCR.

In one embodiment, the transcriptional regulatory domain may an activating transcription factor domain which is capable of inducing expression of the nucleic acid encoding the CAR.

The ligand may be an entity which is increased in a tumour microenvironment compared to a non-tumour microenvironment.

In one embodiment, the transcriptional regulatory domain may be a repressor transcription factor domain which is capable of inhibiting expression of the nucleic acid encoding the CAR.

The ligand may be an entity which is reduced in a tumour microenvironment compared to a non-tumour microenvironment.

The ligand may be a metabolite. For example, where the ligand is an entity which is increased in a tumour microenvironment compared to a non-tumour microenvironment the metabolite may be lactate, ornithine, adenosine, inosine, glutamate or kynurenic acid; or where the ligand is an entity which is reduced in a tumour microenvironment compared to a non-tumour microenvironment the metabolite may be tryptophan, glutamine or glucose.

The GPCR may be GPR81, GPR4, GPR68, GPR65, GPRC6A, GRM 1-8 or GPR35.

The GPCR may be GPRC6A, GRM1 or GPR1.

In one embodiment, the ligand may be a small molecule drug.

The protease domain may comprise a Tobacco Etch Virus protease, a furin protease, a tobacco vein mottling virus (TVMV) protease or a plum pox virus Nia protease.

The GPCR-targeting domain may comprise an arrestin domain. For example, the arrestin domain may comprise arrestin 1, arrestin beta 1, arrestin beta 2 or arrestin 3.

The cell may be a T cell.

In another aspect the present invention provides a polynucleotide encoding at least two of the CAR, GPCR and intracellular component according to the first aspect of the present invention.

The polynucleotides may encode a CAR, GPCR and an intracellular component according to the first aspect of the present invention.

The polynucleotide may comprise nucleic acid sequences which are co-expression sites that enable the co-expression of the CAR, GPCR and/or intracellular component as defined in the first aspect of the present invention.

Each co-expression site may be selected from a self-cleaving peptide, a protease cleavage site and an internal ribosome entry site.

In one embodiment, the polynucleotide may comprise the following structure:
iPROM - CAR - cPROM - GPCR - CS - TF- coexpr - TarDomain/Protease
   or
iREP - CAR - cPROM - GPCR - CS - Rep - coexpr - TarDomain/Protease
in which
iPROM is a transcriptional regulatory element which is capable of recruiting TF to induce expression of CAR;
iREP is a transcriptional regulatory element which is capable of recruiting Rep to inhibit expression of CAR;
CAR is a nucleic acid sequence encoding a chimeric antigen receptor;
cPROM is a constitutively active promoter which drives expression of the GPCR and TarDomain/Protease;
GPCR is a nucleic acid sequence encoding a G-protein coupled receptor;
CS is a nucleic acid sequence which comprises a cleavage site for a protease;
TF is a nucleic acid sequence encoding an activating transcription factor domain which is capable of promoting the expression of the nucleic acid encoding the CAR;
Rep is a nucleic acid sequence encoding a repressor domain which is capable of inhibiting the expression of the nucleic acid encoding the CAR;
TarDomain/Protease is a nucleic acid sequence encoding a GPCR-targeting domain linked to a protease domain; and
coexpr is a nucleic acid sequence enabling co-expression of the activating transcription factor domain or repressor domain and the TarDomain/Protease.

The sequence coexpr may encode an amino acid sequence comprising a self-cleaving peptide.

In a further aspect the present invention provides a kit which comprises at least two polynucleotides which between them encode a CAR, GPCR and intracellular component as defined in the first aspect of the present invention.

The CAR, GPCR and intracellular component may each be encoded by a separate polynucleotide.

The kit may comprise:
(i) a polynucleotide which comprises the following structure:
   cPROM - GPCR - CS - TF- coexpr - TarDomain/Protease
   and
(ii) a polynucleotide which comprises the following structure:
   iPROM - CAR
or
(i) a polynucleotide which comprises the following structure:
   cPROM - GPCR - CS - Rep - coexpr - TarDomain/Protease
   and
(ii) a polynucleotide which comprises the following structure:
   iREP - CAR
in which
cPROM is a constitutively active promoter which drives expression of the GPCR and TarDomain/Protease
GPCR is a nucleic acid sequence encoding a G-protein coupled receptor
CS is a nucleic acid sequence which comprises a cleavage site for the protease
TF is a nucleic acid sequence encoding an activating transcription factor domain which is capable of promoting the expression of the nucleic acid encoding the CAR
Rep is a nucleic acid sequence encoding a repressor domain which is capable of inhibiting the expression of the nucleic acid encoding the CAR
TarDomain/Protease is a nucleic acid sequence encoding a GPCR-targeting domain and a protease
coexpr is a nucleic acid sequence enabling co-expression of the transcription factor domain or repressor domain and the TarDomain/Protease;
iPROM is a transcriptional regulatory element which is capable of recruiting TF to induce expression of CAR
iREP is a transcriptional regulatory element which is capable of recruiting Rep to inhibit expression of CAR; and
CAR is a nucleic acid sequence encoding a chimeric antigen receptor.

In a further aspect the present invention provides a vector comprising a polynucleotide according to present invention.

In another aspect the present invention provides a kit comprising a plurality of vectors each comprising a polynucleotide according to the present invention.

The vectors may beintegrating viral vectors or transposons.

In another aspect the present invention provides a kit which comprises a polynucleotide or a plurality of polynucleotides, or a vector or a plurality of vectors according to the present invention and a small molecule drug which is capable of binding to the GPCR.

In another aspect the present invention relates to method for making a cell according to the first aspect of the present invention, which comprises the step of introducing a polynucleotide or a plurality of polynucleotides or a vector or a plurality of vectors according to the present invention into the cell.

The cell may be from a sample isolated from a subject.

In another aspect the present invention relates to a pharmaceutical composition which comprises a cell, a polynucleotide or a vector or plurality of vectors according to the present invention.

In a further aspect the present invention relates to a pharmaceutical composition according to the present invention for use in treating and/or preventing a disease in a subject.

The pharmaceutical composition may comprise:
cells that have been isolated from a subject and transduced or transfected with a polynucleotide or a plurality of polynucleotides or a vector or a plurality of vectors according to the present invention.

The pharmaceutical composition may be administered in combination with a small molecule drug which binds to the GPCR in order to induce expression of a CAR according to the present invention.

The progression of disease and/or toxic activity in the subject may be monitored and the dose of small molecule drug may be adjusted to provide acceptable levels of disease progression and/or toxic activity.

The pharmaceutical composition may be administered in combination with a small molecule drug which binds to the GPCR in order to inhibit expression of a CAR according to the present invention.

The toxic activity in the subject may be monitored and a small molecule drug may be administered which is capable of binding to the GPCR to reduce adverse toxic effects.

The progression of disease and/or toxic activity in the subject may be monitored and a small molecule drug may be administered which is capable of binding to the GPCR to provide acceptable levels of disease progression and/or toxic activity.

The disease may be cancer.

The present GPCR modulated CAR signalling system offers an improvement over previous systems, such as those described by Roybal *et al.* (as above), because the GPCRs are capable of recognising soluble ligands in the tumour microenvironment - for example 'tumour enriched' or 'tumour depleted' metabolites - rather than surface expressed tumour antigens. As such, the activation of the present CAR signalling systems can be controlled by a general tumour microenvironment rather than by the expression of a specific combination of cell surface tumour antigens.

### DESCRIPTION OF THE FIGURES

**Figure 1** - Chimeric Antigen Receptors
   a) Schematic diagram illustrating a classical CAR. (b) to (d): Different generations and permutations of CAR endodomains: (b) initial designs transmitted ITAM signals alone through FcεR1-γ or CD3ζ endodomain, while later designs transmitted additional (c) one or (d) two co-stimulatory signals in the same compound endodomain.

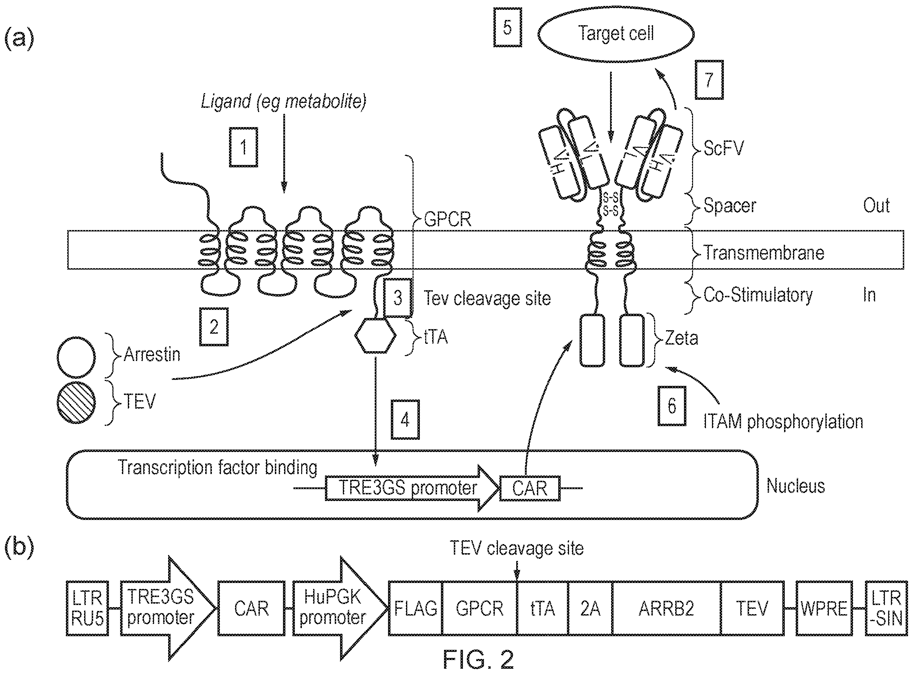

**Figure 2** - Illustrative GPCR CAR transcriptional activation signalling system (AND gate)
   (a) [1] Ligand (e.g. metabolite) binds to the GPCR, leading to receptor phosphorylation. [2] Beta-arrestin-TEV fusion protein is recruited to cytoplasmic tail of the GPCR. [3] TEV cleaves the cytoplasmic tail of the GPCR, releasing the transcription factor (tTa/activating transcription factor). [4] The transcription factor binds to the upstream activating sequence (UAS), leading to transcription of CAR. [5] Surface-expressed CAR engages CD19+ target. [6] Zeta is phosphorylated and signal, leading to killing of the target cell. (b) Cassette design to facilitate the approach shown in Panel A. A SIN lentiviral vector is used, upon integration the 5' LTR is inactive. A conditionally active promoter drives expression of the CAR. A constitutively active promoter drives expression of the GPCR / Arrestin components. These are co-expressed using the FMD-2A sequence. A woodchuck pre-processing element follows the final reading frame and the 3' LTR acts as a polyadenylation sequence.

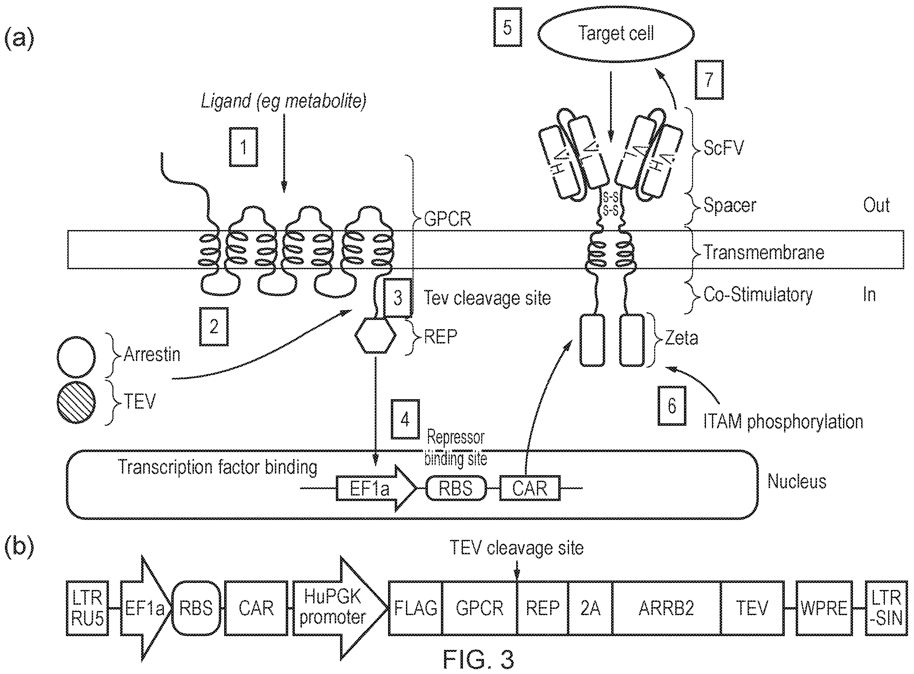

**Figure 3** - Illustrative GPCR CAR transcriptional repression signalling system (AND NOT gate)
   (a) [1] Ligand (e.g. metabolite) binds to the GPCR, leading to receptor phosphorylation. [2] Beta-arrestin-TEV fusion protein is recruited to the cytoplasmic tail of GPCR. [3] TEV cleaves the cytoplasmic tail of GPCR, releasing the repressor (REP). [4] The repressor binds to the transcriptional start site and inhibits transcription of CAR. [5] Surface-expressed CAR now fails to engage target. [6] There is no CAR to activate. The target cell is spared. (b) Cassette design to facilitate the approach shown in Panel A. A SIN lentiviral vector is used, upon integration the 5' LTR is inactive. A promoter drives expression of the CAR. A repressor binding sequence is inserted at or close to the transcriptional start site. A constitutively active promoter drives expression of the GPCR / Arrestin components. These are co-expressed using the FMD-2A sequence. A woodchuck pre-processing element follows the final reading frame and the 3' LTR acts as a polyadenylation sequence.

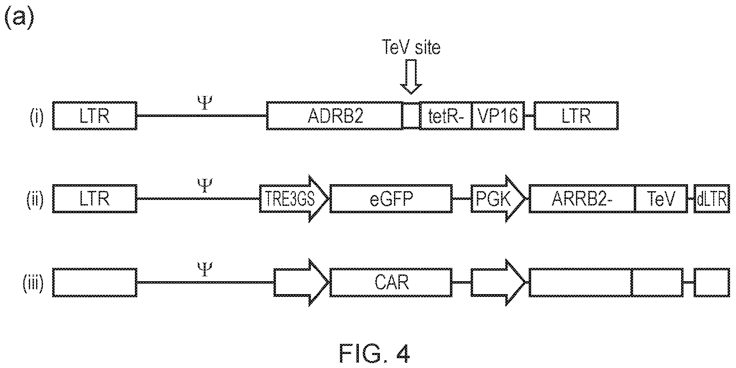

**Figure 4** - Maps of different vectors generated; (i) ADRB2 fused to a tetR-VP16 transcription factor via a TeV cleavage site is expressed in a retroviral vector; (ii) A self-inactivating retroviral vector expresses eGFP from the Tre3GS promoter. A second down-stream internal promoter PGK drives constitutive expression of a fusion between ARRB2 and TeV. (iii) A third retroviral vector is identical to the second except a CD19 CAR is expressed under TRE3GS promoter instead of eGFP.

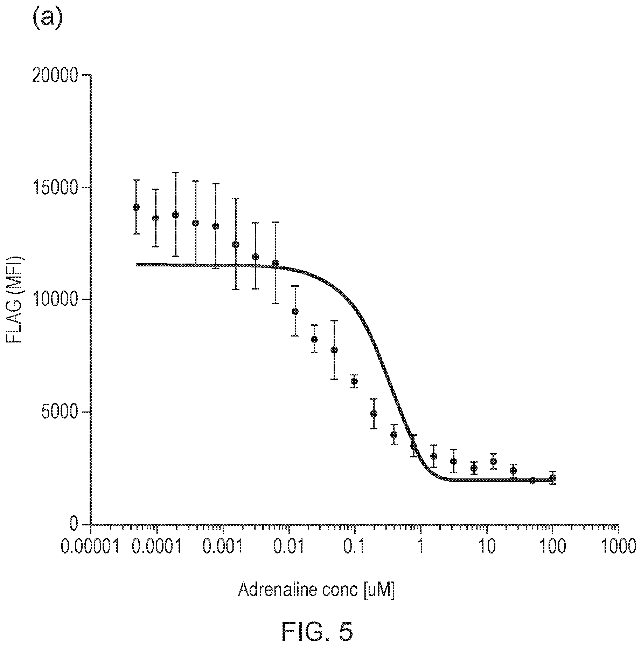

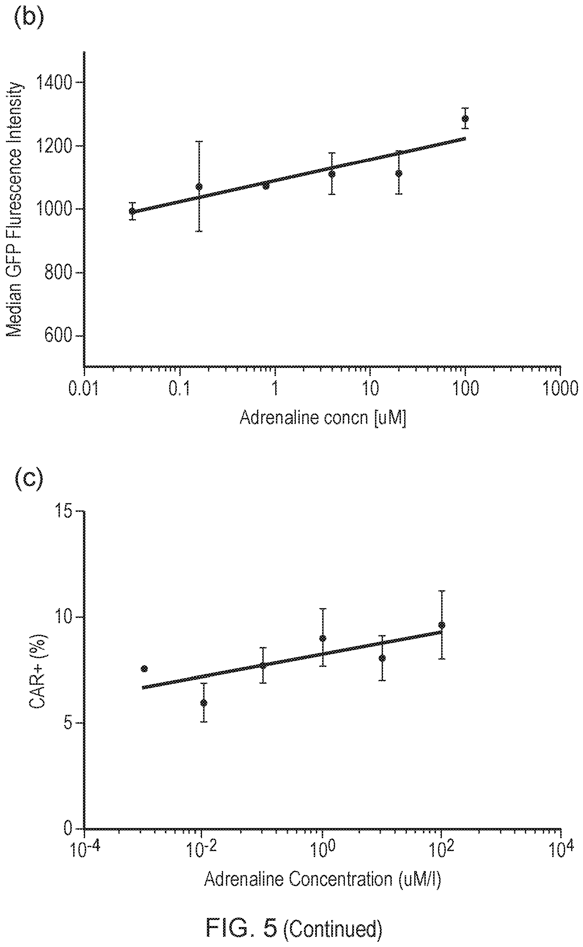

Figure 5 - (a) Down-regulation of FLAG epitope tag in response to adrenaline; (b) Upregulation of eGFP in response to adrenaline; (c) upregulation of CAR in response to adrenaline.

### DETAILED DESCRIPTION OF THE INVENTION

As used herein, the singular forms "a", "an", and "the" include both singular and plural referents unless the context clearly dictates otherwise.

The terms "comprising", "comprises" and "comprised of as used herein are synonymous with "including", "includes" or "containing", "contains", and are inclusive or open-ended and do not exclude additional, non-recited members, elements or method steps. The terms "comprising", "comprises" and "comprised of also include the term "consisting of'.

### MODULATING

The use of ligand recognition by a GPCR to modulate the expression level of a reporter gene is known in the art (Kroeze et al.; 2015; Nature Structural & Molecular Biology; 22, 362-369). Such assays are typically used for drug discovery and identifying ligands for orphan GPCRs.

The term "modulating" is used herein to mean that the binding of a ligand to the GPCR alters the level of CAR expression compared to the level of CAR expression in the absence of binding of ligand to the GPCR.

In one embodiment ligand binding by the GPCR may induce or augment expression of the CAR. As such, CAR expression and productive CAR signalling is only possible in the presence of the GPCR ligand. Upon antigen binding to the CAR, such signalling results in cell activation, for example triggering T cell activation and target cell killing.

In one embodiment, when a ligand binds to the GPCR, the GPCR is phosphorylated as part of the induced signalling cascade. An intracellular component comprising a protease domain linked to a GPCR-targeting domain is then recruited to phosphorylated cytoplasmic tail of GPCR via an interaction between the GPCR-targeting domain and an intracellular domain of the GPCR. The protease domain then cleaves the GPCR at a protease cleavage site, releasing a activating transcription factor domain from the membrane-associated GPCR. The activating transcription factor then translocates to the nucleus and induces or promotes transcription of a polynucleotide encoding the CAR via recruitment to a transcriptional regulatory element. The CAR polypeptide is then expressed at the surface of the cell and can activate the cell upon antigen binding.

An illustration of this embodiment is shown in Figure 2.

Binding of ligand to the GPCR may result in CAR expression levels which are at least 2, 5, 10, 50, 100, 1,000 or 10,000-fold higher than the corresponding CAR expression level in the absence of ligand binding to the GPCR.

In one embodiment, ligand binding by the GPCR may inhibit or reduce expression of the CAR. As such, CAR expression and productive CAR signalling is only possible in the absence of the GPCR ligand.

In one embodiment, when a ligand binds to the GPCR, the GPCR is phosphorylated as part of the induced signalling cascade. An intracellular component comprising a protease domain linked to a GPCR-targeting domain is then recruited to the phosphorylated cytoplasmic tail of GPCR via an interaction between the GPCR-targeting domain and an intracellular domain of the GPCR. The protease domain then cleaves the GPCR at a protease cleavage site, releasing a repressor transcription factor domain from the membrane-associated GPCR. The repressor transcription factor domain then translocates to the nucleus and inhibits or represses the transcription of a polynucleotide encoding the CAR via recruitment to a transcriptional regulatory element. As such, the CAR polypeptide is not expressed at the surface of the cell and cannot bind antigen.

An illustration of this embodiment is shown in Figure 3.

Binding of ligand to the GPCR may result in CAR expression levels which are at least 2, 5, 10, 50, 100, 1,000 or 10,000-fold lower than the corresponding CAR expression level in the absence of ligand binding to the GPCR.

The expression level of the CAR may be determined by methods which are known in the art, for example RT-qPCR or flow cytometry.

Signalling through a CAR may be determined by a variety of methods known in the art. Such methods include assaying signal transduction, for example assaying levels of specific protein tyrosine kinases (PTKs), breakdown of phosphatidylinositol 4,5-biphosphate (PIP₂), activation of protein kinase C (PKC) and elevation of intracellular calcium ion concentration. Functional readouts, such as clonal expansion of T cells, upregulation of activation markers on the cell surface, differentiation into effector cells and induction of cytotoxicity or cytokine secretion may also be utilised. As an illustration, levels of interleukin-2 (IL-2) produced by cells of the present invention may be determined in the presence or absence of binding of antigen to the CAR in the presence of varying concentrations of the GPCR ligand.

### G-PROTEIN COUPLED-RECEPTOR (GPCR)

GPCRs are a large class of multi-span transmembrane proteins which recognize and respond to a broad range of small molecules.

GPCRs may also be referred to as seven-transmembrane domain receptors, 7TM receptors, heptahelical receptors, serpentine receptor, and G protein-linked receptors (GPLR). Thus GPCR comprises a plurality of linked transmembrane domains.

GPCRs are characterized by an extracellular N-terminus, followed by seven transmembrane α-helices connected by three intracellular and three extracellular loops, and finally an intracellular C-terminus.

The protease cleavage site linked to a transcriptional regulatory domain as described herein may be present at any of the GPCR intracellular domains. In a preferred embodiment the protease cleavage site linked to a transcriptional regulatory domain is present at the intracellular C-terminus of the GPCR.

When a ligand binds to the GPCR it causes a conformational change in the GPCR, which allows it to act as a guanine nucleotide exchange factor (GEF). The GPCR can then activate an associated G protein by exchanging its bound GDP for a GTP. The G protein's α subunit, together with the bound GTP, can then dissociate from the β and γ subunits to further affect intracellular signaling proteins or target functional proteins directly depending on the α subunit type (Gαs, Gαi/o, Gαq/11, Gα12/13).

The two principal signal transduction pathways downstream of GPCRs are the cAMP signal pathway and the phosphatidylinositol signal pathway.

In addition to G-Proteins, a common feature of GPCRs is their recruitment of arrestins which normally reside in the cytosol to the active phosphorylated GPCR at the membrane.

The GPCR may be any suitable GPCR which is capable of binding a ligand. For example, the GPCR may be a GPCR which is capable of binding a metabolite which is enriched or depleted in a tumour microenvironment or a GPCR which is capable of binding a small molecule drug.

GPCR libraries and methods for identifying ligands for orphan GPCRs are available (see Kroeze *et al.*; as above). In addition, it is possible to engineer a GPCR to be specific for a desired ligand, for example as described in Ault et al. (Protein Engineering, Design and Selection 19, 1-8 (2006)).

### LIGAND

Cancer is a genetically complex and heterogenous group of conditions. However, a pathological hallmark of malignant transformation is disordered metabolism in the tumour microenvironment.

As used herein, the term "tumour microenvironment" may refer to the proximal environment of the malignant and non-transformed cells that comprise a tumour. For example, apart from malignant cells, the tumour microenvironment may comprise cells of the immune system, the tumour vasculature and lymphatics, as well as fibroblasts, pericytes and adipocytes. The tumour microenvironment also includes the extracellular matrix of the tumour (see Balkwill et al; 2012; Journal of Cell Science 125, 5591-5596).

The most well-known manifestation of this disordered metabolism of the tumour microenvironment is known as the 'Warburg effect'. Cancer cells typically rely on 'aerobic glycolysis' to support their proliferation and anabolic growth - even in the presence of ample oxygen. This phenomenon has been demonstrated across multiple tumour types and is now regarded as a key metabolic characteristic of cancer (Farreira; 2010; Molecular Pathology; 89: 372-380).

By way of example, during the process of aerobic glycolysis, ATP is rapidly generated by the preferential catabolism of glucose to lactate, rather than full metabolism to carbon dioxide via mitochondrial oxidative phosphorylation. As a result, lactate production is increased, resulting in elevated lactate concentrations in the tumour microenvironment (Yamagata et al.; 1998; Br. J. Cancer; 77; 1726-1731).

Further evidence for the metabolic dysregulation of cancer comes from a systematic study performed using CORE profiling which revealed both global and lineage-specific metabolic signatures across a range of 60 human cancer cell lines: for example, leukaemia-derived cell lines released ornithine while adenosine and inosine were released from melanoma cells. Cell lines with high proliferative rates were demonstrated to be dependent on exogenous glycine for growth (Jain et al; 2012; Science; 336; 1040-1044).

In one embodiment, the GPCR ligand may be a metabolite. The term "metabolite" is used herein according to its usual meaning to refer to a small molecule which is produced as an intermediate and/or product of metabolism.

In one embodiment the ligand may be an entity which is increased in a tumour microenvironment compared to a non-tumour microenvironment. As used herein, "increased in a tumour microenvironment" refers to entities which are enriched in a tumour microenvironment compared to a non-tumour microenvironment. For example, an entity which is enriched in a tumour microenvironment may be present at a 10, 20, 50, 100, 500 or 1000-fold greater level in a tumour microenvironment compared to a non-tumour microenvironment.

There are many examples of GPCRs which can be useful in the recognition of metabolic disturbances associated with cancer. For instance, a cell surface receptor for lactate has been described, termed GPR81 (HCA1) (Ge et al; 2008; The Journal of Lipid Research; 49; 797-803). GPR81 is expressed predominantly in adipocytes and skeletal muscle, where activation by lactate results in reduced conversion of ATP to cAMP and thereby reduction of lipolysis. Receptor activation is mediated by lactate with half-maximal concentrations of approximately 5mM, which is the range of 5-20mM described in many cancers (Yamagata *et al;* as above).

An example HCA1 is the HCA1 having the UniProtKB accession number Q9BXC0 and shown as SEQ ID NO: 1.

SEQ ID NO: 1
MYNGSCCRIEGDTISQVMPPLLIVAFVLGALGNGVALCGFCFHMKTWKPSTVYLFNLAVADFLLMICLPFRTDYYLR
RRHWAFGDIPCRVGLFTLAMNRAGSIVFLTVVAADRYFKVVHPHHAVNTISTRVAAGIVCTLWALVILGTVYLLLEN
HLCVQETAVSCESFIMESANGWHDIMFQLEFFMPLGIILFCSFKIVWSLRRRQQLARQARMKKATRFIMVVAIVFIT
CYLPSVSARLYFLWTVPSSACDPSVHGALHITLSFTYMNSMLDPLVYYFSSPSFPKFYNKLKICSLKPKQPGHSKTQ
RPEEMPISNLGRRSCISVANSFQSQSDGQWDPHIVEWH

Examples of metabolites which may be enriched in a tumour microenvironment include, but are not limited to; lactate, ornithine, adenosine, inosine, glutamate and kynurenic acid.

A further consequence of the Warburg effect is that tumours typically maintain an acidic environment, due to the presence of lactate which serves as the proton donor (Yang et al; 2013; Front Physiol.; 4:354). A group of GPCRs which are activated by an acidic microenvironment include GPR4, GPR68 and GPR65. Protonation of extracellular histidine residues of these receptors leads to downstream signalling and utilisation of such acid-sensitive GPCRs in the present signalling system enables productive CAR signalling only in a tumour-specific microenvironment.

Amino acid sequences for GPR4, GPR68 and GPR65 are shown as SEQ ID NO: 2-4
SEQ ID NO: 2 
MGNHTWEGCHVDSRVDHLFPPSLYIFVIGVGLPTNCLALWAAYRQVQQRNELGVYLMNLSIADLLYICTLPLWVDYF
LHHDNWIHGPGSCKLFGFIFYTNIYISIAFLCCISVDRYLAVAHPLRFARLRRVKTAVAVSSVVWATELGANSAPLF
HDELFRDRYNHTFCFEKFPMEGWVAWMNLYRVFVGFLFPWALMLLSYRGILRAVRGSVSTERQEKAKIKRLALSLIA
IVLVCFAPYHVLLLSRSAIYLGRPWDCGFEERVFSAYHSSLAFTSLNCVADPILYCLVNEGARSDVAKALHNLLRFL
ASDKPQEMANASLTLETPLTSKRNSTAKAMTGSWAATPPSQGDQVQLKMLPPAQ
SEQ ID NO: 3 
MGNITADNSSMSCTIDHTIHQTLAPVVYVTVLVVGFPANCLSLYFGYLQIKARNELGVYLCNLTVADLFYICSLPFW
LQYVLQHDNWSHGDLSCQVCGILLYENIYISVGFLCCISVDRYLAVAHPFRFHQFRTLKAAVGVSVVIWAKELLTSI
YFLMHEEVIEDENQHRVCFEHYPIQAWQRAINYYRFLVGFLFPICLLLASYQGILRAVRRSHGTQKSRKDQIQRLVL
STVVIFLACFLPYHVLLLVRSVWEASCDFAKGVFNAYHFSLLLTSFNCVADPVLYCFVSETTHRDLARLRGACLAFL
TCSRTGRAREAYPLGAPEASGKSGAQGEEPELLTKLHPAFQTPNSPGSGGFPTGRLA
SEQ ID NO: 4

MNSTCIEEQHDLDHYLFPIVYIFVIIVSIPANIGSLCVSFLQAKKESELGIYLFSLSLSDLLYALTLPLWIDYTWNK
DNWIFSPALCKGSAFLMYMNFYSSTAFLTCIAVDRYLAVVYPLKFFFLRTRRFALMVSLSIWILETIFNAVMLWEDE
TVVEYCDAEKSNFTLCYDKYPLEKWQINLNLFRTCTGYAIPLVTILICNRKVYQAVRHNKATENKEKKRIIKLLVSI
TVTFVLCFTPFHVMLLIRCILEHAVNFEDHSNSGKRTYTMYRITVALTSLNCVADPILYCFVTETGRYDMWNILKFC
TGRCNTSQRQRKRILSVSTKDTMELEVLE

The GPCR may comprise a variant of SEQ ID NO: 1-4 which retains the functional activity of the corresponding amino acid sequence shown as SEQ ID NO: 1-4. The variant may share at least 70, 75, 80, 85, 90, 95, 98 or 99% sequence identity with one of SEQ ID NO: 1 - 4.

Examples of GPCRs which bind to entities which are enriched in a tumour microenvironment or are activated by conditions of the tumour microenvironment are shown in Table 1 (illustrative UniProt accession numbers are shown in brackets).

**Table 1**

| **GPCR** | **Ligand / Activation Mechanism** |
|---|---|
| GPR81/HCA1 (Q9BXC0) | Lactate |
| GPR4(P46093), GPR68 (Q15743), GPR65 (Q8IYL9) | Acidic pH |
| GPRC6A (Q5T6X5) | Extraneous amino acids (e.g. ornithine, adenosine, inosine) |
| GRM1 - GRM8 (GRM1 - Q13255) | Glutamate |
| GPR35 (Q9HC97) | Kynurenic acid |

In one embodiment the ligand may be an entity which is reduced in a tumour microenvironment compared to a non-tumour microenvironment. As used herein, "reduced in a tumour microenvironment" refers to entities which are depleted in a tumour microenvironment compared to a non-tumour microenvironment. For example, an entity which is reduced in a tumour microenvironment may be present at a 10, 20, 50, 100, 500 or 1000-fold lower level in a tumour microenvironment compared to a non-tumour microenvironment.

Tumour microenvironments are known to be deficient in certain metabolites. For instance, the enzyme Indoleamine 2,3-dioxygenase (IDO) is over-expressed by many cancers. This enzyme converts tryptophan into metabolites such as kynuremine and 3-hydroxyanthranilic acid and a cancer microenvironment could hence be sensed as one which is low in tryptophan (see e.g. Munn et al. (J. Clin. Invest. 117, 1147-1154 (2007)).

Examples of entities which may be reduced in a tumour microenvironment include, but are not limited to, tryptophan, glutamine and glucose.

Examples of GPCRs which bind to entities which are depleted in a tumour microenvironment are shown in Table 2.

**Table 2**

| **GPCR** | **Ligand** / **Activation Mechanism** |
|---|---|
| GPRC6A (Q5T6X5) | Tryptophan |
| GRM1 (Q13255) | Glutamine |
| GPR1 (Q12361) | Glucose |

### SMALL MOLECULE DRUG

In one embodiment the GPCR ligand may be a small molecule drug.

"Small molecule drug" is used herein according to its usual meaning to refer to a pharmaceutical molecule with a low molecular weight (e.g. less than 900 daltons) and with a size in the order of 10⁻⁹ m which binds to a specific GPCR target.

Examples of small molecule drugs and the GPCRs they bind are shown in Table 3.

**Table 3**

| **GPCR** | **Small molecule drug** |
|---|---|
| GPR3 (P46089) | Diphenyleneiodonium chloride (DPI) |
| hM4Dl* | Clozapine-N-oxide |
| hM3Dq* | Clozapine-N-oxide |

| | |
|---|---|
| *see Becnel et al.; Cell Reports 4(5): 1049-1059, 2013 | |

### TRANSCRIPTIONAL REGULATORY DOMAIN

As used herein the term "transcriptional regulatory domain" is used to refer to a factor which is capable of modulating the expression of a polynucleotide following recruitment to a transcriptional regulatory element.

Transcriptional regulatory domains typically comprise of sub-domains which include a DNA binding sub-domain and either a transcriptional activator or a repressor. Such regulatory domains may be naturally occuring or may be generated artificicially, typicially by fusions of protein domains. Such domains increase or repress transcription of a gene containing the cognate sequence of the DNA binding domain.

The transcriptional regulatory domain may be an activating transcription factor domain which induces expression of a polynucleotide following recruitment to a transcriptional regulatory element.

The transcriptional regulatory domain may be a repressor transcription factor domain which inhibits expression of a polynucleotide following recruitment to a transcriptional regulatory element.

DNA binding domains recognize a specific DNA sequence or set of DNA sequences. In addition to naturally occurring DNA binding domains with an inherient specificity, DNA binding domains can be artificially generated which recognize any desired DNA sequence. Such artificial DNA binding domains include artificial zinc-finger domains or Transcription activator-effector like nucleases (TALENs). Other strategies to generate artificial DNA binding domains include co-expression of CrispR/CAS9 with a short guide mRNA.

An example of a DNA binding domain is the GAL4 DNA binding domain (SEQ ID NO: 5) which recognizes the upstream activating sequence (UAS) (SEQ ID NO: 6).
SEQ ID NO: 5 - GAL4 
MRKLLSSIEQACDICRLKKLKCSKEKPKCAKCLKNNWECRYSPKTKRSPLTRAHLTEVESRLERLEQLFL
LIFPREDLDMILKMDSLQDIKALLTGLFVQDNVNKDAVTDRLASVETDMPLTLRQHRISATSSSEESSNK
GQRQLTV
SEQ ID NO: 6 - UAS
   CGG-N₁₁-CCG
Wherein N is any nucleotide

The DNA binding domain may comprise a variant of SEQ ID NO: 5 which has at least 80%, 85%, 90%, 95%, 98% or 99% sequence identity to SEQ ID NO: 5 provided that the sequence is capable of binding to the UAS.

By way of example, transcriptional activators include - but are not limited to - VP16 and p65.
SEQ ID NO: 7 - VP16 
APPTDVSLGDELHLDGEDVAMAHADALDDFDLDMLGDGDSPGPGFTPHDSAPYGALDMADFEFEQMFTDA
LGIDEYGG
SEQ ID NO: 8 - p65

By way of example, transcriptional repressor include - but are not limited to the Tetracycline repressor (TetR), SSX3 and V-ErbA.
SEQ ID NO: 9 - SSX3 
MNGDDTFARRPTVGAQIPEKIQKAFDDIAKYFSKEEWEKMKVSEKIVYVYMKRKYEAMTKLGFKAILPSF
MRNKRVTDFQGNDFDNDPNRGNQVQRPQMTFGRLQGIFPKIMPKKPAEEGNVSKEVPEASGPQNDGKQLC
PPGKPTTSEKINMISGPKRGEHAWTHRLRERKQLVIYEEISDPEEDDE
SEQ ID NO: 10 - V-Erb-A oncoprotein

METVIKVISSAPVVAMPVVIKTEGPAWTPLEPEDTRWLDGKHKRKSSQCLVKSSMSGYIPSCLDKDEQCV
VCGDKPTGYHYRCITCEGCKSFFRRTIQKNLHPTYSCTYDGCCVIDKITRNQCQLCRFKKCISVGMAMDL
VLDDSKRVAKRKLIEENRERRRKEEMIKSLQHRPSPSAEEWELIHVVTEAHRSTNAQGSHWKQRRKFLLE
DIGQSPMASMLDGDKVDLEAFTEFTKIITPAITRVVDFAKNLPMFSELPCEDQIILLKGCCMEIMSLRAA
VRYDPESETLTLSGEMAVKREQLKNGGLGVVSDAIFDLGKSLSAFNLDDTEVALLQAVLLMSSDRTGLIC
VDKIEKCQESYLLAFEHYINYRKHNIPHFWSKLLMKVADLRMIGAYHASRFLHMKVECPTELSPQEVGPD
HCMKCAHFIDGPHCVKACPAGVLGENDTLVWKYADANAVCQLCHPNCTRGCKGPGLEGCPNGSKTPSIAA
GVVGGLLCLVVVGLGIGLYLRRRHIVRKRTLRRLLQERELVEPLTPSGEAPNQAHLRILKETEFKKVKVL
GFGAFGTVYKGLWIPEGEKVTIPVAIKELREATSPKANKEILDEAYVMASVDNPHVCRLLGICLTSTVQL
ITQLMPYGCLLDYIREHKDNIGSQYLLNWCVQIAKGMNYLEERHMVHRDLAARNVLVKTPQHVKITDFGL
AKQLGADEKEYHAEGGKVPIKWMALESILHRIYTHQSDVWSYGVTVWELMTFGSKPYDGIPASEISSVLE
KGERLPQPPICTIDVYMIMVKCWMSGADSRPKFRELIAEFSKMARDPPRYLVIQGDERMHLPSPTDSKFY
RTLMEEEDMEDIVDADEYLVPHQGFFNSPSTSRTPLLSSLSATSNNSATKCIDRNGGHPVREDGFLPAPE
YVNQLMPKKPSTAMVQNQIYNYISLTAISKLPMDSRYQNSHSTAVDNPEYLE

The TetR is an example of a repressor domain which contains both DNA recognition and transcriptional repression elements (SEQ ID NO: 11). The DNA binding domain of TetR is shown as SEQ ID NO: 12. TetR acts on the Tetracycline response element DNA sequence (SEQ ID NO: 13).
SEQ ID NO: 11 - Tetracycline repressor (TetR) 
RLDKSKVINSALELLNEVGIEGLTTRKLAQKLGVEQPTLYWHVKNKRALLDALAIEMLDRHHTHFCPLEG
ESWQDFLRNNAKSFRCALLSHRDGAKVHLGTRPTEKQYETLENQLAFLCQQGFSLENALYALSAVGHFTL
GCVLEDQEHQVAKEERETPTTDSMPPLLRQAIELFDHQGAEPAFLFGLELIICGLEKQLKCESGS
SEQ ID NO: 12 - TetR DNA binding domain
   TTRKLAQKLGVEQPTLYWHV
SEQ ID NO: 13 - Tetracycline response element (TRE3GS promoter)

GAGTTTACTCCCTATCAGTGATAGAGAACGTATGAAGAGTTTACTCCCTATCAGTGATAGAGAACGTATG
CAGACTTTACTCCCTATCAGTGATAGAGAACGTATAAGGAGTTTACTCCCTATCAGTGATAGAGAACGTA
TGACCAGTTTACTCCCTATCAGTGATAGAGAACGTATCTACAGTTTACTCCCTATCAGTGATAGAGAACG
TATATCCAGTTTACTCCCTATCAGTGATAGAGAACGTATAAGCTTTGCTTATGTAAACCAGGGCGCCTAT
AAAAGAGTGCTGATTTTTTGAGTAAACTTCAATTCCACAACACTTTTGTCTTATACCAACTTTCCGTACC
ACTTCCTACCCTCGTAAA

The transcriptional activator or repressor may comprise a variant of any one of SEQ ID NO: 7 - 11 which shares at least 80%, 85%, 90%, 95%, 98% or 99% sequence identity to SEQ ID NO: 7 to 11 provided that the sequence provides an effective transcriptional activator or transcriptional repressor domain as described herein.

In a preferred embodiment, the transcriptional regulatory domain comprises a nuclear localisation signal (NLS). The NLS may be or comprise the SV40 Large T-antigen NLS, the nucleoplasmin NLS, the EGL-13 NLS, the c-Myc NLS or the TUS-protein NLS.

The NLS may be or comprise a sequence shown as SEQ ID NO: 14 to 18 or a variant thereof.
SEQ ID NO: 14 (SV40 Large T-antigen NLS) - PKKKRKV
SEQ ID NO: 15 (nucleoplasmin NLS) - AVKRPAATKKAGQAKKKKLD
SEQ ID NO: 16 (EGL-13 NLS) - MSRRRKANPTKLSENAKKLAKEVEN
SEQ ID NO: 17 (c-Myc NLS) - PAAKRVKLD
SEQ ID NO: 18 (TUS-protein NLS) - KLKIKRPVK

A variant sequence may have at least 80%, 85%, 90%, 95%, 98% or 99% sequence identity to SEQ ID NO: 14 to 18 provided that the sequence provides an effective NLS which is capable promoting translocation of the transcription factor domain to the nucleus as described herein.

### NUCLEIC ACID ENCODING A CAR

As described herein, once the transcriptional regulatory domain is released from the GPCR by the action of the protease domain of the intracellular component, it translocates to the nucleus where it regulates the expression of a nucleic acid encoding a CAR.

The CAR may be any CAR as described herein.

'Regulates the expression of a nucleic acid encoding a CAR' as used herein may mean that the transcriptional regulatory domain is recruited to a nucleic acid sequence via an interaction between the DNA binding domain of the transcriptional regulatory domain. The recruiting nucleic acid sequence is proximal to, and typically positioned upstream of, the nucleic acid sequence which encodes the CAR.

Once recruited to the nucleic acid sequence via the DNA binding domain, the transcriptional regulatory domain then enhances or represses the transcription of the CAR - depending on whether the transcriptional regulatory domain comprises a transcriptional activator or a transcriptional repressor domain as described herein.

As such, the DNA binding domain of the transcriptional regulatory domain should be capable of binding to a recruiting nucleic acid sequence which is proximal to the nucleic acid sequence encoding the CAR. Suitable DNA binding domains and recruiting nucleic sequences are well known in the art and include, but are not limited to, the GAL4 DNA binding domain (SEQ ID NO: 5) which recognizes the UAS (SEQ ID NO: 6) and the TetR DNA binding domain (SEQ ID NO: 12) which recognizes the tetracycline response element sequence (SEQ ID NO: 13).

### INTRACELLULAR COMPONENT

The present intracellular component comprises a protease domain linked to a GPCR-targeting domain. The intracellular component is a soluble polypeptide which is recruited to the intracellular domain of the GPCR following binding of ligand to the GPCR.

As such, the intracellular component is capable of co-localising with a GPCR which has bound, or is bound, by a ligand; but is not capable of co-localising with a GPCR which has not bound, or is not bound, by a ligand.

As used herein, the term "co-localising" is analogous to ligation/recruitment of the GPCR-targeting domain to the GPCR and is intended to mean that the GPCR-targeting domain is preferentially recruited to the GPCR following the binding of a ligand to the GPCR.

### ARRESTIN

In one embodiment, the GPCR-targeting domain may comprise an arrestin domain.

Arrestins are a small family of proteins important for regulating signal transduction at G protein-coupled receptors.

In response to a stimulus, GPCRs activate heterotrimeric G proteins. In order to turn off this response, or adapt to a persistent stimulus, active receptors need to be desensitized. The first step is phosphorylation by a class of serine/threonine kinases called G protein coupled receptor kinases (GRKs). GRK phosphorylation specifically prepares the activated receptor for arrestin binding. Arrestin binding to the receptor blocks further G protein-mediated signalling and targets receptors for internalization, and redirects signalling to alternative G protein-independent pathways, such as β-arrestin signalling. In addition to GPCRs, arrestins bind to other classes of cell surface receptors and a variety of other signalling proteins.

Mammals express four arrestin subtypes and each arrestin subtype is known by multiple aliases.

Arrestin-1 was originally identified as the S-antigen (SAG) causing uveitis (autoimmune eye disease), then independently described as a 48 kDa protein that binds light-activated phosphorylated rhodopsin. An example human arrestin-1 is the human arrestin-1 having the UniProtKB accession number P10523 and shown as SEQ ID NO: 19.

SEQ ID NO: 19







Arrestin beta 1 was the first non-visual arrestin cloned. It was first named β-arrestin because between two GPCRs available in purified form at the time, rhodopsin and β2-adrenergic receptor, it showed preference for the latter. An example human arrestin beta 1 is the human arrestin beta 1 having the UniProtKB accession number P49407 and shown as SEQ ID NO: 20.

SEQ ID NO: 20
MGDKGTRVFKKASPNGKLTVYLGKRDFVDHIDLVDPVDGVVLVDPEYLKERRVYVTLTCAFRYGREDLDVLGLTFRK
DLFVANVQSFPPAPEDKKPLTRLQERLIKKLGEHAYPFTFEIPPNLPCSVTLQPGPEDTGKACGVDYEVKAFCAENL
EEKIHKRNSVRLVIRKVQYAPERPGPQPTAETTRQFLMSDKPLHLEASLDKEIYYHGEPISVNVHVTNNTNKTVKKI
KISVRQYADICLFNTAQYKCPVAMEEADDTVAPSSTFCKVYTLTPFLANNREKRGLALDGKLKHEDTNLASSTLLRE
GANREILGIIVSYKVKVKLVVSRGGLLGDLASSDVAVELPFTLMHPKPKEEPPHREVPENETPVDTNLIELDTNDDD
IVFEDFARQRLKGMKDDKEEEEDGTGSPQLNNR

Arrestin beta 2 was the second non-visual arrestin cloned and was first termed β-arrestin-2 (retroactively changing the name of β-arrestin into β-arrestin-1). An example human arrestin beta 2 is the human arrestin beta 2 having the UniProtKB accession number P32121 and shown as SEQ ID NO: 21.

SEQ ID NO: 21
MGEKPGTRVFKKSSPNCKLTVYLGKRDFVDHLDKVDPVDGVVLVDPDYLKDRKVFVTLTCAFRYGREDLDVLGLSFR
KDLFIATYQAFPPVPNPPRPPTRLQDRLLRKLGQHAHPFFFTIPQNLPCSVTLQPGPEDTGKACGVDFEIRAFCAKS

LEEKSHKRNSVRLVIRKVQFAPEKPGPQPSAETTRHFLMSDRSLHLEASLDKELYYHGEPLNVNVHVTNNSTKTVKK
IKVSVRQYADICLFSTAQYKCPVAQLEQDDQVSPSSTFCKVYTITPLLSDNREKRGLALDGKLKHEDTNLASSTIVK
EGANKEVLGILVSYRVKVKLVVSRGGDVSVELPFVLMHPKPHDHIPLPRPQSAAPETDVPVDTNLIEFDTNYATDDD
IVFEDFARLRLKGMKDDDYDDQLC

Arrestin-3 is also known as cone arrestin, after photoreceptor type that expresses it, and X-arrestin, after the chromosome where its gene resides. An example human arrestin-4 is the human arrestin-4 having the UniProtKB accession number P36575 and shown as SEQ ID NO: 22.

SEQ ID NO: 22
MSKVFKKTSSNGKLSIYLGKRDFVDHVDTVEPIDGVVLVDPEYLKCRKLFVMLTCAFRYGRDDLEVIGLTFRKDLYV
QTLQVVPAESSSPQGPLTVLQERLLHKLGDNAYPFTLQMVTNLPCSVTLQPGPEDAGKPCGIDFEVKSFCAENPEET
VSKRDYVRLVVRKVQFAPPEAGPGPSAQTIRRFLLSAQPLQLQAWMDREVHYHGEPISVNVSINNCTNKVIKKIKIS
VDQITDVVLYSLDKYTKTVFIQEFTETVAANSSFSQSFAVTPILAASCQKRGLALDGKLKHEDTNLASSTIIRPGMD
KELLGILVSYKVRVNLMVSCGGILGDLTASDVGVELPLVLIHPKPSHEAASSEDIVIEEFTRKGEEESQKAVEAEGD
EGS

The arrestin domain may comprise an amino acid sequence shown as SEQ ID NO: 19 - 22.

In a preferred embodiment the arrestin domain comprises an amino acid sequence shown as SEQ ID NO: 19 or a variant thereof.

The arrestin domain may comprise a variant of SEQ ID NO: 19 - 22 which retains the functional activity of the corresponding amino acid sequence shown as SEQ ID NO: 19 - 22; i.e. - the ability to bind to an activated GPCR as described herein.

The variant may comprise a sequence which shares at least 70%, at least 75%, at least 80%, at least 85%, at least 90%, at least 95% or at least 99% sequence identity with SEQ ID NO: 19 and retains the functional activity of the amino acid sequence shown as SEQ ID NO: 19.

The variant may comprise a sequence which shares at least 70%, at least 75%, at least 80%, at least 85%, at least 90%, at least 95% or at least 99% sequence identity with SEQ ID NO: 20 and retains the functional activity of the amino acid sequence shown as SEQ ID NO: 20.

The variant may comprise a sequence which shares at least 70%, at least 75%, at least 80%, at least 85%, at least 90%, at least 95% or at least 99% sequence identity with SEQ ID NO: 21 and retains the functional activity of the amino acid sequence shown as SEQ ID NO: 21.

The variant may comprise a sequence which shares at least 70%, at least 75%, at least 80%, at least 85%, at least 90%, at least 95% or at least 99% sequence identity with SEQ ID NO: 22 and retains the functional activity of the amino acid sequence shown as SEQ ID NO: 22.

The variant may be a fragment of any of SEQ ID NO: 19-22 or a variant thereof which retains the ability to bind to an activated GPCR as described herein. The fragment may be, for example, less than 400, 350, 300, 250, 200, 150, 100 or 50 amino acids provided that it retains the ability to bind to an activated GPCR as described herein.

### LINKED

As used herein, the term "linked" refers to two or more connected polypeptides, wherein each polypeptide provides a separate functional activity. Typically, the term linked is used to refer to two connected polypeptides.

The term linked encompasses direct and indirect linkage. A direct linkage implies a direct covalent binding between the first and second polypeptides without an intervening linker sequence. An indirect linkage implies that the first and second polypeptides polypeptide are linked by intervening amino acid sequences

### PROTEASE DOMAIN AND PROTEASE CLEAVAGE SITE

The protease domain may, for example, be or comprise a Tobacco Etch Virus protease, a furin protease, a tobacco vein mottling virus (TVMV) protease or a plum pox virus Nia protease.

The protease domain may be or comprise a Tobacco Etch Virus protease and the cleavage site may be a Tobacco Etch Virus (TEV) cleavage site.

TEV protease is a highly sequence-specific cysteine protease which is chymotrypsin-like proteases. It is very specific for its target cleavage site and is therefore frequently used for the controlled cleavage of fusion proteins both in vitro and in vivo. The consensus TEV cleavage site is ENLYFQ\S (SEQ ID NO: 23) (where '\' denotes the cleaved peptide bond). Mammalian cells, such as human cells, do not express TEV protease.

An illustrative TEV protease is the protein shown as SEQ ID NO: 24.

SEQ ID NO: 24 – TEV Protease

SLFKGPRDYNPISSTICHLTNESDGHTTSLYGIGFGPFIITNKHLFRRNNGTLLVQSLHGVFKVKNTTTL
QQHLIDGRDMIIIRMPKDFPPFPQKLKFREPQREERICLVTTNFQTKSMSSMVSDTSCTFPSSDGIFWKH
WIQTKDGQCGSPLVSTRDGFIVGIHSASNFTNTNNYFTSVPKNFMELLTNQEAQQWVSGWRLNADSVLWG
GHKVFMSKPEEPFQPVKEATQLMNELVYSQ

The protease domain may be or comprise a furin protease and the cleavage site may be a furin cleavage site.

Furin is an enzyme which belongs to the subtilisin-like proprotein convertase family. The members of this family are proprotein convertases that process latent precursor proteins into their biologically active products. Furin is a calcium-dependent serine endoprotease that can efficiently cleave precursor proteins at their paired basic amino acid processing sites. Examples of furin substrates include proparathyroid hormone, transforming growth factor beta 1 precursor, proalbumin, pro-beta-secretase, membrane type-1 matrix metalloproteinase, beta subunit of pro-nerve growth factor and von Willebrand factor. Furin cleaves proteins just downstream of a basic amino acid target sequence (canonically, Arg-X-(Arg/Lys)-Arg') (SEQ ID NO: 25) and is enriched in the Golgi apparatus.

An illustrative furin protein is the human furin protein having Uniprot accession number P09958 and shown as SEQ ID NO: 26.

SEQ ID NO: 26

MELRPWLLWVVAATGTLVLLAADAQGQKVFTNTWAVRIPGGPAVANSVARKHGFLNLGQIFGDYYHFWHR
GVTKRSLSPHRPRHSRLQREPQVQWLEQQVAKRRTKRDVYQEPTDPKFPQQWYLSGVTQRDLNVKAAWAQ
GYTGHGIVVSILDDGIEKNHPDLAGNYDPGASFDVNDQDPDPQPRYTQMNDNRHGTRCAGEVAAVANNGV
CGVGVAYNARIGGVRMLDGEVTDAVEARSLGLNPNHIHIYSASWGPEDDGKTVDGPARLAEEAFFRGVSQ
GRGGLGSIFVWASGNGGREHDSCNCDGYTNSIYTLSISSATQFGNVPWYSEACSSTLATTYSSGNQNEKQ
IVTTDLRQKCTESHTGTSASAPLAAGIIALTLEANKNLTWRDMQHLVVQTSKPAHLNANDWATNGVGRKV
SHSYGYGLLDAGAMVALAQNWTTVAPQRKCIIDILTEPKDIGKRLEVRKTVTACLGEPNHITRLEHAQAR
LTLSYNRRGDLAIHLVSPMGTRSTLLAARPHDYSADGFNDWAFMTTHSWDEDPSGEWVLEIENTSEANNY
GTLTKFTLVLYGTAPEGLPVPPESSGCKTLTSSQACVVCEEGFSLHQKSCVQHCPPGFAPQVLDTHYSTE
NDVETIRASVCAPCHASCATCQGPALTDCLSCPSHASLDPVEQTCSRQSQSSRESPPQQQPPRLPPEVEA
GQRLRAGLLPSHLPEVVAGLSCAFIVLVFVTVFLVLQLRSGFSFRGVKVYTMDRGLISYKGLPPEAWQEE
CPSDSEEDEGRGERTAFIKDQSAL

The protease domain may be or comprise a tobacco vein mottling virus (TVMV) protease and the cleavage site may be a TVMV protease cleavage site.

The tobacco vein mottling virus (TVMV) protease is a close relative of TEV protease with a distinct cleavage site sequence specificity (ETVRFQG/S) (SEQ ID NO: 27).

An illustrative TVMV protease is the protein shown as SEQ ID NO: 28.

SEQ ID NO: 28 – TMVM protease

SKALLKGVRDFNPISACVWLLENSSDGHSERLFGIGFGPYIIANQHLFRRNNGELTIKTMHGEFKVKNST
QLQMKPVEGRDIIVIKMAKDFPPFPQKLKFRQPTIKDRVCMVSTNFQQKSVSSLVSESSHIVHKEDTSFW
QHWITTKDGQCGSPLVSIIDGNILGIHSLTHTTNGSNYFVEFPEKFVATYLDAADGWCKNWKFNADKISW
GSFTLVEDAPEDDFMAKKTVAAIMDDLVRTQ

The protease domain may be or comprise a plum pox virus Nia protease and the cleavage site may be a plum pox virus Nia protease cleavage site.

SEQ ID NO: 29 – plum pox virus Nia protease

QFWDGFTNSFMQCKLRETDHQCTSDLDVKECGYVAAIVCQAIIPCGKITCLQCAQKYSYMSQQEIRDRFS
TVIEQHEKTVMDNYPQFSHVLAFLKRYRELMRVENQNYEALKDITHMIGERKEAPFSHLNKINELIIKGG
MMSAQDYMEASNCLRELARYQKNRTENIQSGSIKAFRNKISAKAYVNMQLMCDNQLDTNGNFVWGQREYH
AKRFFRNYFDVIDTSEGYRRHIVRENPRGTRKLAIGNLVMSTNLAALRRQLLGEECTNFDVSKECTSKRG
ENFVYQCCCVTHEDGTPLKSEIISPTKNHLVIGNSGDSKYVDLPKTDKGGMYIAKAGYCYINVFLAMLVN
VNESEAKSFTKTVRDTLVPKLGLWPSMMDLATACHFLAVLYPETRNAELPRILVDHESKLFHVVDSYGSL
STGLHILKANTVNQLISFASDTLDSSMKMYLVG

The protease domain may be or comprise a sequence shown as SEQ ID NO: 24, 26, 28 or 29 or a variant thereof. A variant sequence may have at least 80%, 85%, 90%, 95%, 98% or 99% sequence identity to SEQ ID NO: 24, 26, 28 or 29 provided that the sequence provides an protease domain which capable of cleaving at the corresponding cleavage site.

### VARIANT

Sequence comparisons can be conducted by eye, or more usually, with the aid of readily available sequence comparison programs. These publicly and commercially available computer programs can calculate sequence identity between two or more sequences.

Sequence identity may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % sequence identity therefore firstly requires the production of an optimal alignment, taking into consideration gap penalties. A suitable computer program for carrying out such an alignment is the GCG Wisconsin Bestfit package (University of Wisconsin, U.S.A; Devereux et al., 1984, Nucleic Acids Research 12:387). Examples of other software than can perform sequence comparisons include, but are not limited to, the BLAST package (see Ausubel *et al.,* 1999 *ibid -* Chapter 18), FASTA (Atschul et al., 1990, J. Mol. Biol., 403-410) and the GENEWORKS suite of comparison tools. Both BLAST and FASTA are available for offline and online searching (see Ausubel *et al.,* 1999 *ibid,* pages 7-58 to 7-60). However it is preferred to use the GCG Bestfit program.

Although the final sequence identity can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % sequence identity. The software typically does this as part of the sequence comparison and generates a numerical result.

The terms "variant" according to the present invention includes any substitution of, variation of, modification of, replacement of, deletion of or addition of one (or more) amino acids from or to the sequence providing the resultant amino acid sequence retains substantially the same activity as the unmodified sequence.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

It will be understood by a skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described here to reflect the codon usage of any particular host organism in which the polypeptides are to be expressed.

A nucleic acid sequence or amino acid sequence as described herein may comprise, consist of or consist essentially of a nucleic acid sequence or amino acid sequence as shown herein.

### CHIMERIC ANTIGEN RECEPTORS (CARs)

CARs, which are shown schematically in Figure 1, are chimeric type I trans-membrane proteins which connect an extracellular antigen-recognizing domain (binder) to an intracellular domain (endodomain). The binder is typically a single-chain variable fragment (scFv) derived from a monoclonal antibody (mAb), but it can be based on other formats which comprise an antibody-like antigen binding site. A spacer domain is usually necessary to isolate the binder from the membrane and to allow it a suitable orientation. A common spacer domain used is the Fc of IgG1. More compact spacers can suffice e.g. the stalk from CD8α and even just the IgG1 hinge alone, depending on the antigen. A trans-membrane domain anchors the protein in the cell membrane and connects the spacer to the endodomain.

Early CAR designs had endodomains derived from the intracellular parts of either the γ chain of the FcεR1 or CD3ζ. Consequently, these first generation receptors transmitted immunological signal 1, which was sufficient to trigger T-cell killing of cognate target cells but failed to fully activate the T-cell to proliferate and survive. To overcome this limitation, compound endodomains have been constructed: fusion of the intracellular part of a T-cell co-stimulatory molecule to that of CD3ζ results in second generation receptors which can transmit an activating and co-stimulatory signal simultaneously after antigen recognition. The co-stimulatory domain most commonly used is that of CD28. This supplies the most potent co-stimulatory signal - namely immunological signal 2, which triggers T-cell proliferation. Some receptors have also been described which include TNF receptor family endodomains, such as the closely related OX40 and 41BB which transmit survival signals. Even more potent third generation CARs have now been described which have endodomains capable of transmitting activation, proliferation and survival signals.

CAR-encoding nucleic acids may be transferred to T cells using, for example, retroviral vectors. Lentiviral vectors may be employed. In this way, a large number of cancer-specific T cells can be generated for adoptive cell transfer. When the CAR binds the target-antigen, this results in the transmission of an activating signal to the T-cell it is expressed on. Thus the CAR directs the specificity and cytotoxicity of the T cell towards tumour cells expressing the targeted antigen.

The present CAR comprises: (i) an antigen-binding domain; (ii) a spacer; (iii) a transmembrane domain; and (iv) an intracellular domain.

### SIGNAL PEPTIDE

The CAR may comprise a signal peptide so that when the CAR is expressed inside a cell, such as a T-cell, the nascent protein is directed to the endoplasmic reticulum and subsequently to the cell surface, where it is expressed.

The core of the signal peptide may contain a long stretch of hydrophobic amino acids that has a tendency to form a single alpha-helix. The signal peptide may begin with a short positively charged stretch of amino acids, which helps to enforce proper topology of the polypeptide during translocation. At the end of the signal peptide there is typically a stretch of amino acids that is recognized and cleaved by signal peptidase. Signal peptidase may cleave either during or after completion of translocation to generate a free signal peptide and a mature protein. The free signal peptides are then digested by specific proteases.

The signal peptide may be at the amino terminus of the molecule.

The signal peptide may comprise the SEQ ID NO: 30, 31 or 32 or a variant thereof having 5, 4, 3, 2 or 1 amino acid mutations (insertions, substitutions or additions) provided that the signal peptide still functions to cause cell surface expression of the CAR.
SEQ ID NO: 30: MGTSLLCWMALCLLGADHADG

The signal peptide of SEQ ID NO: 30 is compact and highly efficient. It is predicted to give about 95% cleavage after the terminal glycine, giving efficient removal by signal peptidase.
SEQ ID NO: 31: MSLPVTALLLPLALLLHAARP

The signal peptide of SEQ ID NO: 31 is derived from IgG1.
SEQ ID NO: 32 MAVPTQVLGLLLLWLTDARC

The signal peptide of SEQ ID NO: 32 is derived from CD8.

### ANTIGEN BINDING DOMAIN

The antigen binding domain is the portion of the CAR which recognizes antigen. Numerous antigen-binding domains are known in the art, including those based on the antigen binding site of an antibody, antibody mimetics, and T-cell receptors. For example, the antigen-binding domain may comprise: a single-chain variable fragment (scFv) derived from a monoclonal antibody; a natural ligand of the target antigen; a peptide with sufficient affinity for the target; a single domain antibody; an artificial single binder such as a Darpin (designed ankyrin repeat protein); or a single-chain derived from a T-cell receptor.

Various tumour associated antigens (TAA) are known, as shown in the following Table 4. The antigen-binding domain used in the present invention may be a domain which is capable of binding a TAA as indicated therein.

**Table 4**

| **Cancer type** | **TAA** |
|---|---|
| Diffuse Large B-cell Lymphoma | CD19, CD20, CD22 |
| Breast cancer | ErbB2, MUC1 |
| AML | CD13, CD33 |
| Neuroblastoma | GD2, NCAM, ALK, GD2 |
| B-CLL | CD19, CD52, CD160 |
| Colorectal cancer | Folate binding protein, CA-125 |
| Chronic Lymphocytic Leukaemia | CD5, CD19 |
| Glioma | EGFR, Vimentin |
| Multiple myeloma | BCMA, CD138 |
| Renal Cell Carcinoma | Carbonic anhydrase IX, G250 |
| Prostate cancer | PSMA |
| Bowel cancer | A33 |

### SPACER DOMAIN

CARs comprise a spacer sequence to connect the antigen-binding domain with the transmembrane domain and spatially separate the antigen-binding domain from the endodomain. A flexible spacer allows the antigen-binding domain to orient in different directions to facilitate binding.

Examples of amino acid sequences for these spacers are given below:
SEQ ID NO: 33 (hinge-CH₂CH₃ of human lgG1) 
AEPKSPDKTHTCPPCPAPPVAGPSVFLFPPKPKDTLMIARTPEVTCVVVDVSHEDPEVKFNWYVDGVEVH
NAKTKPREEQYNSTYRVVSVLTVLHQDWLNGKEYKCKVSNKALPAPIEKTISKAKGQPREPQVYTLPPSR
DELTKNQVSLTCLVKGFYPSDIAVEWESNGQPENNYKTTPPVLDSDGSFFLYSKLTVDKSRWQQGNVFSC
SVMHEALHNHYTQKSLSLSPGKKD
SEQ ID NO: 34 (human CD8 stalk)
   TTTPAPRPPTPAPTIASQPLSLRPEACRPAAGGAVHTRGLDFACDI
SEQ ID NO: 35 (human IgG1 hinge)
   AEPKSPDKTHTCPPCPKDPK
SEQ ID NO: 36 (CD2 ectodomain) 
KEITNALETWGALGQDINLDIPSFQMSDDIDDIKWEKTSDKKKIAQFRKEKETFKEKDTYKLFKNGTLKI
KHLKTDDQDIYKVSIYDTKGKNVLEKIFDLKIQERVSKPKISWTCINTTLTCEVMNGTDPELNLYQDGKH
LKLSQRVITHKWTTSLSAKFKCTAGNKVSKESSVEPVSCPEKGLD
SEQ ID NO: 37 (CD34 ectodomain)

SLDNNGTATPELPTQGTFSNVSTNVSYQETTTPSTLGSTSLHPVSQHGNEATTNITETTVKFTSTSVITS
VYGNTNSSVQSQTSVISTVFTTPANVSTPETTLKPSLSPGNVSDLSTTSTSLATSPTKPYTSSSPILSDI

KAEIKCSGIREVKLTQGICLEQNKTSSCAEFKKDRGEGLARVLCGEEQADADAGAQVCSLLLAQSEVRPQ
CLLLVLANRTEISSKLQLMKKHQSDLKKLGILDFTEQDVASHQSYSQKT

The spacer may be a variant of any of SEQ ID NO: 33 to 37 which shares at least 70%, at least 75%, at least 80%, at least 85%, at least 90%, at least 95% or at least 99% sequence identity with SEQ ID NO: 33 to 37 and retains the functional activity of the amino acid sequence shown as SEQ ID NO: 33 to 37.

### TRANSMEMBRANE DOMAIN

The transmembrane domain is the sequence of the CAR that spans the membrane.

A transmembrane domain may be any protein structure which is thermodynamically stable in a membrane. This is typically an alpha helix comprising of several hydrophobic residues. The transmembrane domain of any transmembrane protein can be used to supply the transmembrane portion of the invention. The presence and span of a transmembrane domain of a protein can be determined by those skilled in the art using the TMHMM algorithm (http://www.cbs.dtu.dk/services/TMHMM-2.0/). Further, given that the transmembrane domain of a protein is a relatively simple structure, i.e a polypeptide sequence predicted to form a hydrophobic alpha helix of sufficient length to span the membrane, an artificially designed TM domain may also be used (US 7052906 B1 describes synthetic transmembrane components).

The transmembrane domain may be derived from CD28, which gives good receptor stability.

The transmembrane domain may be derived from any type I transmembrane protein. The transmembrane domain may be a synthetic sequence predicted to form a hydrophobic helix.

The transmembrane domain may comprise the sequence shown as SEQ ID NO: 38.
SEQ ID NO: 38 (CD28 transmembrane domain)
FWVLVWGGVLACYSLLVTVAFIIFWV

### INTRACELLULAR DOMAIN

The intracellular domain of a classical CAR is the signalling domain. After antigen recognition, receptors cluster, native CD45 and CD148 are excluded from the synapse and a signal is transmitted to the cell. The most commonly used endodomain component is that of CD3-zeta which contains three immunoreceptor tyrosine-based activation motifs (ITAMs). This transmits an activation signal to the cell after antigen is bound. CD3-zeta may not provide a fully competent activation signal and additional co-stimulatory signalling may be needed. For example, chimeric CD28 and OX40 can be used with CD3-Zeta to transmit a proliferative / survival signal, or all three can be used together.

The intracellular domain may comprise a single signalling endodomain. The signalling domain may comprise a single endodomain selected from CD3 zeta endodomain, CD28 endodomain, 41BB endodomain and an OX40 endodomain

The intracellular domain may comprise the CD3-Zeta endodomain alone.

The intracellular domain may comprise the sequence shown as SEQ ID NO: 39 to 42 or a variant thereof having at least 80% sequence identity.
SEQ ID NO: 39 - CD3 Z endodomain 
RVKFSRSADAPAYQQGQNQLYNELNLGRREEYDVLDKRRGRDPEMGGKPRRKNPQEGLYNELQKDKMAEA
YSEIGMKGERRRGKGHDGLYQGLSTATKDTYDALHMQALPPR
SEQ ID NO: 40 - CD28 endodomain
   KRSRLLHSDYMNMTPRRPGPTRKHYQPYAPPRDFAAY
SEQ ID NO: 41 - OX40 endodomain
   RRDQRLPPDAHKPPGGGSFRTPIQEEQADAHSTLAKI
SEQ ID NO: 42 - 41BB endodomain
   KRGRKKLLYIFKQPFMRPVQTTQEEDGCSCRFPEEEEGGCEL

The intracellular domain may comprise a plurality of constituent signalling endodomains. By way of example, each intracellular signalling domain may comprise two, three or four or more signalling endodomains. The combination of multiple signalling domains is also referred to herein as a compound signalling domain.

The intracellular domain may comprise the CD3-Zeta endodomain together with any one of CD28, 41BB or OX40. The intracellular domain may comprise the CD3-Zeta endodomain, the CD28 endodomain and the 41BB domain. The intracellular domain may comprise the CD3-Zeta endodomain, the CD28 endodomain and the OX40 endodomain.

The intracellular domain may comprise the sequence shown as SEQ ID NO: 43 to 45 or a variant thereof having at least 80% sequence identity.
SEQ ID NO: 43 - CD28 and CD3 Zeta endodomains 
SKRSRLLHSDYMNMTPRRPGPTRKHYQPYAPPRDFAAYRSRVKFSRSADAPAYQQGQNQLYNELNLGRRE
EYDVLDKRRGRDPEMGGKPRRKNPQEGLYNELQKDKMAEAYSEIGMKGERRRGKGHDGLYQGLSTATKDT
YDALHMQALPPR
SEQ ID NO: 44 - CD28, OX40 and CD3 Zeta endodomains 
SKRSRLLHSDYMNMTPRRPGPTRKHYQPYAPPRDFAAYRSRDQRLPPDAHKPPGGGSFRTPIQEEQADAH
STLAKIRVKFSRSADAPAYQQGQNQLYNELNLGRREEYDVLDKRRGRDPEMGGKPRRKNPQEGLYNELQK
DKMAEAYSEIGMKGERRRGKGHDGLYQGLSTATKDTYDALHMQALPPR
SEQ ID NO: 45 - 41BB, OX40 and CD3 Zeta endodomains

KRGRKKLLYIFKQPFMRPVQTTQEEDGCSCRFPEEEEGGCELRRDQRLPPDAHKPPGGGSFRTPIQEEQA
DAHSTLAKIRVKFSRSADAPAYQQGQNQLYNELNLGRREEYDVLDKRRGRDPEMGGKPRRKNPQEGLYNE
LQKDKMAEAYSEIGMKGERRRGKGHDGLYQGLSTATKDTYDALHMQALPPR

A variant sequence may have at least 80%, 85%, 90%, 95%, 98% or 99% sequence identity to SEQ ID NO: 43 to 45 provided that the sequence provides an effective intracellular signalling domain.

### POLYNUCLEOTIDE

As used herein, the terms "polynucleotide", "nucleotide", and "nucleic acid" are intended to be synonymous with each other.

It will be understood by a skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described here to reflect the codon usage of any particular host organism in which the polypeptides are to be expressed.

Nucleic acids according to the invention may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the use as described herein, it is to be understood that the polynucleotides may be modified by any method available in the art. Such modifications may be carried out in order to enhance the in vivo activity or life span of polynucleotides of interest.

The terms "variant", "homologue" or "derivative" in relation to a nucleotide sequence include any substitution of, variation of, modification of, replacement of, deletion of or addition of one (or more) nucleic acid from or to the sequence.

The polynucleotide of the invention is a polynucleotide which encodes at least two of a CAR, GPCR and intracellular component as described herein. For example, the polynucleotide construct may encode a CAR and a GPCR; a CAR and an intracellular component; a GPCR and an intracellular component or a CAR, a GPCR and an intracellular component as described herein.

The polynucleotide may produce a polypeptide which comprises at least two of a CAR, GPCR and intracellular component as described herein, wherein each component is joined by a cleavage site. The cleavage site may be self-cleaving, such that when the polypeptide is produced, it is immediately cleaved into the CAR, GPCR and/or intracellular component without the need for any external cleavage activity.

Various self-cleaving sites are known, including the Foot-and-Mouth disease virus (FMDV) 2a self-cleaving peptide, which has the sequence shown:
SEQ ID NO: 46
   RAEGRGSLLTCGDVEENPGP
   or
SEQ ID NO: 47
   QCTNYALLKLAGDVESNPGP

A 'self-cleaving peptide' refers to a peptide which functions such that when the polypeptide comprising the targeting component and the signalling component and the self-cleaving peptide is produced, it is immediately "cleaved" or separated into distinct and discrete first and second polypeptides without the need for any external cleavage activity.

The self-cleaving peptide may be a 2A self-cleaving peptide from an aphtho- or a cardiovirus. The primary 2A/2B cleavage of the aptho- and cardioviruses is mediated by 2A "cleaving" at its own C-terminus. In apthoviruses, such as foot-and-mouth disease viruses (FMDV) and equine rhinitis A virus, the 2A region is a short section of about 18 amino acids, which, together with the N-terminal residue of protein 2B (a conserved proline residue) represents an autonomous element capable of mediating "cleavage" at its own C-terminus (Donelly et al (2001) as above).

"2A-like" sequences have been found in picornaviruses other than aptho- or cardioviruses, 'picornavirus-like' insect viruses, type C rotaviruses and repeated sequences within Trypanosoma spp and a bacterial sequence (Donnelly et al (2001) as above). The cleavage site may comprise one of these 2A-like sequences, such as:
YHADYYKQRLIHDVEMNPGP (SEQ ID NO: 48)
HYAGYFADLLIHDIETNPGP (SEQ ID NO: 49)
QCTNYALLKLAGDVESNPGP (SEQ ID NO: 50)
ATNFSLLKQAGDVEENPGP (SEQ ID NO: 51)
AARQMLLLLSGDVETNPGP (SEQ ID NO: 52)
RAEGRGSLLTCGDVEENPGP (SEQ ID NO: 53)
TRAEIEDELIRAGIESNPGP (SEQ ID NO: 54)
TRAEIEDELIRADIESNPGP (SEQ ID NO: 55)
AKFQIDKILISGDVELNPGP (SEQ ID NO: 56)
SSIIRTKMLVSGDVEENPGP (SEQ ID NO: 57)
CDAQRQKLLLSGDIEQNPGP (SEQ ID NO: 58)
YPIDFGGFLVKADSEFNPGP (SEQ ID NO: 59)

The co-expressing sequence may be an internal ribosome entry sequence (IRES). The co-expressing sequence may be a protease cleavage site. The co-expressing sequence may be an internal promoter.

By way of example, the polynucleotide may comprise the sequence shown as SEQ ID NO: 60, which is an illustrative nucleic acid of the construct shown in Figure 2(b). This is an example of a transcriptional activator embodiment of the present invention which comprises a HCA1 lactate-sensing GPCR with a TetR-VP16 transcriptional activator which is capable of binding to TRE, and aCD19-CD8STK-41BBz CAR.

SEQ ID NO: 60

GGCCTGAAATAACCTCTGAAAGAGGAACTTGGTTAGGTACCTTCTGAGGCGGAAAGAACCAGCTGTGGAA
TGTGTGTCAGTTAGGGTGTGGAAAGTCCCCAGGCTCCCCAGCAGGCAGAAGTATGCAAAGCATGCATCTC
AATTAGTCAGCAACCAGGTGTGGAAAGTCCCCAGGCTCCCCAGCAGGCAGAAGTATGCAAAGCATGCATC
TCAATTAGTCAGCAACCATAGTCCCTTAAGAATGTAGTCTTATGCAATACTCTTGTAGTCTTGCAACATG
GTAACGATGAGTTAGCAACATGCCTTACAAGGAGAGAAAAAGCACCGTGCATGCCGATTGGTGGAAGTAA
GGTGGTACGATCGTGCCTTATTAGGAAGGCAACAGACGGGTCTGACATGGATTGGACGAACCACTGAATT
GCCGCATTGCAGAGATATTGTATTTAAGTGCCTAGCTCGATACAATAAACGCGCCAGTCCTCCGATTGAC
TGCGTCGCCCGGGTACCCGTATTCCCAATAAAGCCTCTTGCTGTTTGCATCCGAATCGTGGACTCGCTGA
TCCTTGGGAGGGTCTCCTCAGATTGATTGACTGCCCACCTCGGGGGTCTTTCATTTGGAGGTTCCACCGA
GATTTGGAGACCCCTGCCCAGGGACCACCGACCCCCCCGCCGGGAGGTAAGCTGGCCAGCGGTCGTTTCG
TGTCTGTCTCTGTCTTTGGGCGTGTTTGTGCCGGCATCTAGTGTTTGCGCCTGCGTCTGTACTAGTTGGC
TAACTAGATCTGTATCTGGCGGTCCCGCGGAAGAACTGACGAGTTCGTATTCCCGGCCGCAGCCCCTGGG
AGACGTCCCAGCGGCCTCGGGGGCCCGTTTTGTGGCCCATTCTGTATCAGTTAACCTACCCGAGTCGGAC
TTTTTGGAGCTCCGCCACTGTCCGAGGGGTACGTGGCTTTGTTGGGGGACGAGAGACAGAGACACTTCCC
GCCCCCGTCTGAATTTTTGCTTTCGGTTTTACGCCGAAACCGCGCCGCGCGTCTTGTCTGCTGCAGCATC
GTTCTGTGTTGTCTCTGTCTGACTGTGTTTCTGTATTTGTCTGAAAATTAGCGGCCGATCTGACGCGACT
CGAGTTTACTCCCTATCAGTGATAGAGAACGTATGAAGAGTTTACTCCCTATCAGTGATAGAGAACGTAT
GCAGACTTTACTCCCTATCAGTGATAGAGAACGTATAAGGAGTTTACTCCCTATCAGTGATAGAGAACGT
ATGACCAGTTTACTCCCTATCAGTGATAGAGAACGTATCTACAGTTTACTCCCTATCAGTGATAGAGAAC
GTATATCCAGTTTACTCCCTATCAGTGATAGAGAACGTATAAGCTTTGCTTATGTAAACCAGGGCGCCTA

TAAAAGAGTGCTGATTTTTTGAGTAAACTTCAATTCCACAACACTTTTGTCTTATACCAACTTTCCGTAC
CACTTCCTACCCTCGTAAAGTCGACACCATGGAGACCGACACCCTGCTGCTGTGGGTGCTGCTGCTGTGG
GTGCCAGGCAGCACCGGCGAGGTGCAGCTGCAGCAGAGCGGACCCGAGCTGATCAAGCCAGGCGCCAGCG
TGAAGATGAGCTGCAAGGCCAGCGGCTACACCTTCACCAGCTACGTGATGCACTGGGTGAAGCAGAAGCC
AGGCCAGGGCCTGGAGTGGATCGGCTACATCAACCCCTACAACGACGGCACCAAGTACAACGAGAAGTTC
AAGGGCAAGGCCACCCTGACCAGCGACAAGAGCAGCAGCACCGCCTACATGGAGCTGAGCAGCCTGACCA
GCGAGGACAGCGCCGTGTACTACTGCGCCAGAGGCACCTACTACTACGGCAGCCGGGTGTTCGACTACTG
GGGCCAGGGCACCACCCTGACCGTGAGCTCTGGCGGAGGCGGCTCTGGCGGAGGCGGCTCTGGCGGAGGC
GGCAGCGACATCGTGATGACCCAGGCTGCCCCCAGCATCCCCGTGACCCCAGGCGAGAGCGTGAGCATCA
GCTGCCGGAGCAGCAAGAGCCTGCTGAACAGCAACGGCAACACCTACCTGTACTGGTTCCTGCAGCGGCC
AGGCCAGAGCCCCCAGCTGCTGATCTACCGGATGAGCAACCTGGCCAGCGGCGTGCCCGACCGGTTCAGC
GGCAGCGGCAGCGGCACCGCCTTCACCCTGCGGATCAGCCGGGTGGAGGCCGAGGACGTGGGCGTGTACT
ACTGCATGCAGCACCTGGAGTACCCCTTCACCTTCGGAGCCGGCACCAAGCTGGAGCTGAAGCGGTCGGA
TCCCACCACCACCCCAGCCCCACGGCCACCTACCCCTGCCCCAACCATCGCCAGCCAGCCCCTGAGCCTG
CGGCCTGAAGCCTGCAGGCCTGCCGCCGGAGGAGCCGTGCACACAAGGGGCCTGGACTTCGCCTGCGACA
TCTATATCTGGGCCCCCCTGGCCGGGACATGCGGGGTGCTGCTGCTGTCCCTGGTGATTACACTGTATTG
CAAACGGGGCCGGAAGAAGCTGCTGTACATCTTCAAGCAGCCCTTCATGCGGCCCGTGCAGACCACCCAG
GAGGAGGACGGCTGCAGCTGCCGGTTCCCCGAGGAAGAGGAAGGCGGCTGCGAGCTGCGGGTGAAGTTCA
GCCGGAGCGCCGACGCCCCAGCCTACCAGCAGGGCCAGAACCAGCTGTACAACGAGCTGAACCTGGGACG
GCGGGAGGAGTACGACGTGCTGGACAAGCGGCGGGGACGGGACCCCGAGATGGGCGGCAAGCCTCGCCGG
AAGAATCCCCAGGAGGGCCTGTACAACGAGCTGCAGAAGGACAAGATGGCCGAGGCCTACAGCGAGATCG
GCATGAAGGGCGAGCGGCGCCGGGGCAAGGGCCACGACGGCCTGTACCAGGGCCTGAGCACCGCCACCAA
GGACACCTACGACGCCCTGCACATGCAGGCCCTGCCACCCCGGTGAAcgcgtGCCGCTCCGGATTAGTCC
AATTTGTTAAAGACAGGATTCGAGGAGCTTGATAATTCCACGGGGTTGGGGTTGCGCCTTTTCCAAGGCA
GCCCTGGGTTTGCGCAGGGACGCGGCTGCTCTGGGCGTGGTTCCGGGAAACGCAGCGGCGCCGACCCTGG
GTCTCGCACATTCTTCACGTCCGTTCGCAGCGTCACCCGGATCTTCGCCGCTACCCTTGTGGGCCCCCCG
GCGACGCTTCCTGCTCCGCCCCTAAGTCGGGAAGGTTCCTTGCGGTTCGCGGCGTGCCGGACGTGACAAA
CGGAAGCCGCACGTCTCACTAGTACCCTCGCAGACGGACAGCGCCAGGGAGCAATGGCAGCGCGCCGACC
GCGATGGGCTGTGGCCAATAGCGGCTGCTCAGCAGGGCGCGCCGAGAGCAGCGGCCGGGAAGGGGCGGTG
CGGGAGGCGGGGTGTGGGGCGGTAGTGTGGGCCCTGTTCCTGCCCGCGCGGTGTTCCGCATTCTGCAAGC
CTCCGGAGCGCACGTCGGCAGTCGGCTCCCTCGTTGACCGAATCACCGACCTCTCTCCCCAGGGGGATCA
TCGAATTCGCCAACATGCGGCCGCGCCACCATGAAGACGATCATCGCCCTGAGCTACATCTTCTGCCTGG
TATTCGCCGACTACAAGGACGATGATGACGCCAGCATCGATATGTATAATGGCTCCTGTTGCCGGATTGA
AGGCGACACTATCTCTCAAGTAATGCCCCCCCTCCTGATAGTGGCCTTCGTCCTGGGCGCTCTTGGTAAT
GGGGTTGCACTCTGTGGTTTCTGTTTCCACATGAAAACCTGGAAGCCCTCAACAGTGTACCTCTTTAACC
TTGCCGTGGCAGACTTCCTGCTTATGATTTGCCTGCCCTTTAGGACCGACTATTATCTGCGAcGCCGCCA
TTGGGCTTTcGGCGACATCCCCTGTCGGGtTGGTCTGTTTACTCTGGCTATGAATCGGGCCGGCAGTATC
GTCTTTCTCACTGTGGTCGCTGCCGACAGATACTTCAAGGTAGTGCACCCCCACCACGCAGTGAACACAA
TCTCCACTAGAGTTGCAGCTGGAATTGTGTGCACCCTGTGGGCTCTGGTAATCCTGGGCACAGTATACCT
GCTCCTGGAGAATCATTTGTGCGTGCAGGAGACTGCTGTGTCATGTGAATCTTTTATTATGGAGTCCGCA
AACGGGTGGCATGATATCATGTTTCAACTGGAGTTCTTTATGCCCCTTGGCATCATTCTGTTTTGCTCAT
TCAAGATCGTTTGGTCTCTCCGGCGCCGGCAGCAGCTGGCCCGGCAAGCTCGGATGAAAAAGGCCACGCG
CTTTATCATGGTTGTGGCTATCGTCTTCATCACCTGCTACCTTCCTTCCGTGTCCGCAAGACTGTATTTT
CTGTGGACCGTCCCCAGCAGCGCTTGCGATCCCAGTGTCCACGGCGCCCTCCACATCACCTTGAGCTTTA
CGTACATGAACTCTATGCTGGACCCCCTGGTGTACTATTTTAGCTCTCCCTCCTTCCCGAAATTCTATAA
TAAACTTAAGATCTGCAGCCTCAAACCAAAGCAACCAGGCCATTCCAAGACTCAGCGCCCTGAAGAGATG
CCCATTAGCAACCTTGGTAGACGGAGCTGCATCTCCGTCGCGAACTCATTTCAGTCTCAGTCCGACGGAC
AGTGGGACCCACACATTGTTGAGTGGCACATCGATACCGGTGGACGCACCCCACCCAGCCTGGGTCCCCA

AGATGAGTCCTGCACCACCGCCAGCTCCTCCCTGGCCAAGGACACTTCATCGACCGGTGAGAACCTGTAC
TTCCAGCTAAGATTAGATAAAAGTAAAGTGATTAACAGCGCATTAGAGCTGCTTAATGAGGTCGGAATCG
AAGGTTTAACAACCCGTAAACTCGCCCAGAAGCTAGGTGTAGAGCAGCCTACATTGTATTGGCATGTAAA
AAATAAGCGGGCTTTGCTCGACGCCTTAGCCATTGAGATGTTAGATAGGCACCATACTCACTTTTGCCCT
TTAGAAGGGGAAAGCTGGCAAGATTTTTTACGTAATAACGCTAAAAGTTTTAGATGTGCTTTACTAAGTC
ATCGCGATGGAGCAAAAGTACATTTAGGTACACGGCCTACAGAAAAACAGTATGAAACTCTCGAAAATCA
ATTAGCCTTTTTATGCCAACAAGGTTTTTCACTAGAGAATGCATTATATGCACTCAGCGCTGTGGGGCAT
TTTACTTTAGGTTGCGTATTGGAAGATCAAGAGCATCAAGTCGCTAAAGAAGAAAGGGAAACACCTACTA
CTGATAGTATGCCGCCATTATTACGACAAGCTATCGAATTATTTGATCACCAAGGTGCAGAGCCAGCCTT
CTTATTCGGCCTTGAATTGATCATATGCGGATTAGAAAAACAACTTAAATGTGAAAGTGGGTCCGCGTAC
AGCCGCGCGCGTACGAAAAACAATTACGGGTCTACCATCGAGGGCCTGCTCGATCTCCCGGACGACGACG
CCCCCGAAGAGGCGGGGCTGGCGGCTCCGCGCCTGTCCTTTCTCCCCGCGGGACACACGCGCAGACTGTC
GACGGCCCCCCCGACCGATGTCAGCCTGGGGGACGAGCTCCACTTAGACGGCGAGGACGTGGCGATGGCG
CATGCCGACGCGCTAGACGATTTCGATCTGGACATGTTGGGGGACGGGGATTCCCCGGGTCCGGGATTTA
CCCCCCACGACTCCGCCCCCTACGGCGCTCTGGATATGGCCGACTTCGAGTTTGAGCAGATGTTTACCGA
TGCCCTTGGAATTGACGAGTACGGTGGGCAGTGTACTAATTATGCTCTCTTGAAATTGGCTGGAGATGTC
GAGTCCAACCCTGGGCCAATGGGTGAAAAGCCAGGTACCAGGGTCTTCAAGAAGTCGAGCCCTAACTGCA
AGCTCACCGTGTACTTGGGCAAGCGGGACTTCGTAGATCACCTGGACAAAGTGGACCCTGTAGATGGCGT
GGTGCTTGTGGACCCTGACTACCTGAAGGACCGCAAAGTGTTTGTGACCCTCACCTGCGCCTTCCGCTAT
GGCCGTGAAGACCTGGATGTGCTGGGCTTGTCCTTCCGCAAAGACCTGTTCATCGCCACCTACCAGGCCT
TCCCCCCGGTGCCCAACCCACCCCGGCCCCCCACCCGCCTGCAGGACCGGCTGCTGAGGAAGCTGGGCCA
GCATGCCCACCCCTTCTTCTTCACCATACCCCAGAATCTTCCATGCTCCGTCACACTGCAGCCAGGCCCA
GAGGATACAGGAAAGGCCTGCGGCGTAGACTTTGAGATTCGAGCCTTCTGTGCTAAATCACTAGAAGAGA
AAAGCCACAAAAGGAACTCTGTGCGGCTGGTGATCCGAAAGGTGCAGTTCGCCCCGGAGAAACCCGGCCC
CCAGCCTTCAGCCGAAACCACACGCCACTTCCTCATGTCTGACCGCTCCCTGCACCTCGAGGCTTCCCTG
GACAAGGAGCTGTACTACCACGGGGAGCCCCTCAATGTAAATGTCCACGTCACCAACAACTCCACCAAGA
CCGTCAAGAAGATCAAAGTCTCTGTGAGACAGTACGCCGACATCTGCCTCTTCAGCACCGCCCAGTACAA
GTGTCCTGTGGCTCAACTCGAACAAGATGACCAGGTATCTCCCAGCTCCACATTCTGTAAGGTGTACACC
ATAACCCCACTGCTCAGTGACAACCGGGAGAAGCGGGGTCTCGCCCTGGATGGGAAACTCAAGCACGAGG
ACACCAACCTGGCTTCCAGCACCATCGTGAAGGAGGGTGCCAACAAGGAGGTGCTGGGAATCCTGGTGTC
CTACAGGGTCAAGGTGAAGCTGGTGGTGTCTCGAGGCGGGGATGTCTCTGTGGAGCTGCCTTTTGTTCTT
ATGCACCCCAAGCCCCACGACCACATCCCCCTCCCCAGACCCCAGTCAGCCGCTCCGGAGACAGATGTCC
CTGTGGACACCAACCTCATTGAATTTGATACCAACTATGCCACAGATGATGACATTGTGTTTGAGGACTT
TGCCCGGCTTCGGCTGAAGGGGATGAAGGATGACGACTATGATGATCAACTCTGCagcttgtttaaggga
ccacgtgATTACAACCCGATATCGAGCACCATTTGTCATTTGACGAATGAATCTGATGGGCACACAACAT
CGTTGTATGGTATTGGATTTGGTCCCTTCATCATTACAAACAAGCACTTGTTTAGAAGAAATAATGGAAC
ACTGTTGGTCCAATCACTACATGGTGTATTCAAGGTCAAGAACACCACGACTTTGCAACAACACCTCATT
GATGGGAGGGACATGATAATTATTCGCATGCCTAAGGATTTCCCACCATTTCCTCAAAAGCTGAAATTTA
GAGAGCCACAAAGGGAAGAGCGCATATGTCTTGTGACAACCAACTTCCAAACTAAGAGCATGTCTAGCAT
GGTGTCAGACACTAGTTGCACATTCCCTTCATCTGATGGCATATTCTGGAAGCATTGGATTCAAACCAAG
GATGGGCAGTGTGGCAGTCCATTAGTATCAACTAGAGATGGGTTCATTGTTGGTATACACTCAGCATCGA
ATTTCACCAACACAAACAATTATTTCACAAGCGTGCCGAAAAACTTCATGGAATTGTTGACAAATCAGGA
GGCGCAGCAGTGGGTTAGTGGTTGGCGATTAAATGCTGACTCAGTATTGTGGGGGGGCCATAAAGTTTTC
ATGAGCAAACCTGAAGAGCCTTTTCAGCCAGTTAAGGAAGCGACTCAACtcatgaatgaattggtgtact
cgcaatgaGGATCCCCCGGGCTGCAGGAATTCGAGCATCTTACCGCCATTTATACCCATATTTGTTCTGT
TTTTCTTGATTTGGGTATACATTTAAATGTTAATAAAACAAAATGGTGGGGCAATCATTTACATTTTTAG
GGATATGTAATTACTAGTTCAGGTGTATTGCCACAAGACAAACATGTTAAGAAACTTTCCCGTTATTTAC
GCTCTGTTCCTGTTAATCAACCTCTGGATTACAAAATTTGTGAAAGATTGACTGATATTCTTAACTATGT

TGCTCCTTTTACGCTGTGTGGATATGCTGCTTTAATGCCTCTGTATCATGCTATTGCTTCCCGTACGGCT
TTCGTTTTCTCCTCCTTGTATAAATCCTGGTTGCTGTCTCTTTATGAGGAGTTGTGGCCCGTTGTCCGTC
AACGTGGCGTGGTGTGCTCTGTGTTTGCTGACGCAACCCCCACTGGCTGGGGCATTGCCACCACCTGTCA
ACTCCTTTCTGGGACTTTCGCTTTCCCCCTCCCGATCGCCACGGCAGAACTCATCGCCGCCTGCCTTGCC
CGCTGCTGGACAGGGGCTAGGTTGCTGGGCACTGATAATTCCGTGGTGTTGTCGGGGAAGCTGACGTCCT
TTCGAATTCGATATCAAGCTTAACACGAGCCATAGATAGAATAAAAGATTTTATTTAGTCTCCAGAAAAA
GGGGGGAATGAAAGACCCCACCGCTAGCGATATCGAATTCACAACCCCTCACTCGGCGCGCCAGTCCTCC
GACAGACTGAGTCGCCCGGGTACCCGTGTTCTCAATAAACCCTCTTGCAGTTGCATCCGACTCGTGGTCT
CGCTGTTCCTTGGGAGGGTCTCCTCTGAGTGATTGACTGCCCACCTCGGGGGTCTT

By way of further example, the polynucleotide may comprise the sequence shown as SEQ ID NO: 61, which is an illustrative nucleic acid of the construct shown in Figure 3(b). This is an example of a transcriptional repressor embodiment of the present invention which comprises a HCA1 lactate-sensing GPCR with a TetR- V-Erb-A repression domain which is capable of binding to TRE, and aCD19-CD8STK-41BBz CAR.

SEQ ID NO: 61

GGCCTGAAATAACCTCTGAAAGAGGAACTTGGTTAGGTACCTTCTGAGGCGGAAAGAACCAGCTGTGGAA
TGTGTGTCAGTTAGGGTGTGGAAAGTCCCCAGGCTCCCCAGCAGGCAGAAGTATGCAAAGCATGCATCTC
AATTAGTCAGCAACCAGGTGTGGAAAGTCCCCAGGCTCCCCAGCAGGCAGAAGTATGCAAAGCATGCATC
TCAATTAGTCAGCAACCATAGTCCCTTAAGAATGTAGTCTTATGCAATACTCTTGTAGTCTTGCAACATG
GTAACGATGAGTTAGCAACATGCCTTACAAGGAGAGAAAAAGCACCGTGCATGCCGATTGGTGGAAGTAA
GGTGGTACGATCGTGCCTTATTAGGAAGGCAACAGACGGGTCTGACATGGATTGGACGAACCACTGAATT
GCCGCATTGCAGAGATATTGTATTTAAGTGCCTAGCTCGATACAATAAACGCGCCAGTCCTCCGATTGAC
TGCGTCGCCCGGGTACCCGTATTCCCAATAAAGCCTCTTGCTGTTTGCATCCGAATCGTGGACTCGCTGA
TCCTTGGGAGGGTCTCCTCAGATTGATTGACTGCCCACCTCGGGGGTCTTTCATTTGGAGGTTCCACCGA
GATTTGGAGACCCCTGCCCAGGGACCACCGACCCCCCCGCCGGGAGGTAAGCTGGCCAGCGGTCGTTTCG
TGTCTGTCTCTGTCTTTGGGCGTGTTTGTGCCGGCATCTAGTGTTTGCGCCTGCGTCTGTACTAGTTGGC
TAACTAGATCTGTATCTGGCGGTCCCGCGGAAGAACTGACGAGTTCGTATTCCCGGCCGCAGCCCCTGGG
AGACGTCCCAGCGGCCTCGGGGGCCCGTTTTGTGGCCCATTCTGTATCAGTTAACCTACCCGAGTCGGAC
TTTTTGGAGCTCCGCCACTGTCCGAGGGGTACGTGGCTTTGTTGGGGGACGAGAGACAGAGACACTTCCC
GCCCCCGTCTGAATTTTTGCTTTCGGTTTTACGCCGAAACCGCGCCGCGCGTCTTGTCTGCTGCAGCATC
GTTCTGTGTTGTCTCTGTCTGACTGTGTTTCTGTATTTGTCTGAAAATTAGCGGCCGATCTGACGCGACT
CGAGTTTACTCCCTATCAGTGATAGAGAACGTATGAAGAGTTTACTCCCTATCAGTGATAGAGAACGTAT
GCAGACTTTACTCCCTATCAGTGATAGAGAACGTATAAGGAGTTTACTCCCTATCAGTGATAGAGAACGT
ATGACCAGTTTACTCCCTATCAGTGATAGAGAACGTATCTACAGTTTACTCCCTATCAGTGATAGAGAAC
GTATATCCAGTTTACTCCCTATCAGTGATAGAGAACGTATAAGCTTTGCTTATGTAAACCAGGGCGCCTA
TAAAAGAGTGCTGATTTTTTGAGTAAACTTCAATTCCACAACACTTTTGTCTTATACCAACTTTCCGTAC
CACTTCCTACCCTCGTAAAGTCGACACCATGGAGACCGACACCCTGCTGCTGTGGGTGCTGCTGCTGTGG
GTGCCAGGCAGCACCGGCGAGGTGCAGCTGCAGCAGAGCGGACCCGAGCTGATCAAGCCAGGCGCCAGCG
TGAAGATGAGCTGCAAGGCCAGCGGCTACACCTTCACCAGCTACGTGATGCACTGGGTGAAGCAGAAGCC
AGGCCAGGGCCTGGAGTGGATCGGCTACATCAACCCCTACAACGACGGCACCAAGTACAACGAGAAGTTC
AAGGGCAAGGCCACCCTGACCAGCGACAAGAGCAGCAGCACCGCCTACATGGAGCTGAGCAGCCTGACCA
GCGAGGACAGCGCCGTGTACTACTGCGCCAGAGGCACCTACTACTACGGCAGCCGGGTGTTCGACTACTG
GGGCCAGGGCACCACCCTGACCGTGAGCTCTGGCGGAGGCGGCTCTGGCGGAGGCGGCTCTGGCGGAGGC
GGCAGCGACATCGTGATGACCCAGGCTGCCCCCAGCATCCCCGTGACCCCAGGCGAGAGCGTGAGCATCA

GCTGCCGGAGCAGCAAGAGCCTGCTGAACAGCAACGGCAACACCTACCTGTACTGGTTCCTGCAGCGGCC
AGGCCAGAGCCCCCAGCTGCTGATCTACCGGATGAGCAACCTGGCCAGCGGCGTGCCCGACCGGTTCAGC
GGCAGCGGCAGCGGCACCGCCTTCACCCTGCGGATCAGCCGGGTGGAGGCCGAGGACGTGGGCGTGTACT
ACTGCATGCAGCACCTGGAGTACCCCTTCACCTTCGGAGCCGGCACCAAGCTGGAGCTGAAGCGGTCGGA
TCCCACCACCACCCCAGCCCCACGGCCACCTACCCCTGCCCCAACCATCGCCAGCCAGCCCCTGAGCCTG
CGGCCTGAAGCCTGCAGGCCTGCCGCCGGAGGAGCCGTGCACACAAGGGGCCTGGACTTCGCCTGCGACA
TCTATATCTGGGCCCCCCTGGCCGGGACATGCGGGGTGCTGCTGCTGTCCCTGGTGATTACACTGTATTG
CAAACGGGGCCGGAAGAAGCTGCTGTACATCTTCAAGCAGCCCTTCATGCGGCCCGTGCAGACCACCCAG
GAGGAGGACGGCTGCAGCTGCCGGTTCCCCGAGGAAGAGGAAGGCGGCTGCGAGCTGCGGGTGAAGTTCA
GCCGGAGCGCCGACGCCCCAGCCTACCAGCAGGGCCAGAACCAGCTGTACAACGAGCTGAACCTGGGACG
GCGGGAGGAGTACGACGTGCTGGACAAGCGGCGGGGACGGGACCCCGAGATGGGCGGCAAGCCTCGCCGG
AAGAATCCCCAGGAGGGCCTGTACAACGAGCTGCAGAAGGACAAGATGGCCGAGGCCTACAGCGAGATCG
GCATGAAGGGCGAGCGGCGCCGGGGCAAGGGCCACGACGGCCTGTACCAGGGCCTGAGCACCGCCACCAA
GGACACCTACGACGCCCTGCACATGCAGGCCCTGCCACCCCGGTGAAcgcgtGCCGCTCCGGATTAGTCC
AATTTGTTAAAGACAGGATTCGAGGAGCTTGATAATTCCACGGGGTTGGGGTTGCGCCTTTTCCAAGGCA
GCCCTGGGTTTGCGCAGGGACGCGGCTGCTCTGGGCGTGGTTCCGGGAAACGCAGCGGCGCCGACCCTGG
GTCTCGCACATTCTTCACGTCCGTTCGCAGCGTCACCCGGATCTTCGCCGCTACCCTTGTGGGCCCCCCG
GCGACGCTTCCTGCTCCGCCCCTAAGTCGGGAAGGTTCCTTGCGGTTCGCGGCGTGCCGGACGTGACAAA
CGGAAGCCGCACGTCTCACTAGTACCCTCGCAGACGGACAGCGCCAGGGAGCAATGGCAGCGCGCCGACC
GCGATGGGCTGTGGCCAATAGCGGCTGCTCAGCAGGGCGCGCCGAGAGCAGCGGCCGGGAAGGGGCGGTG
CGGGAGGCGGGGTGTGGGGCGGTAGTGTGGGCCCTGTTCCTGCCCGCGCGGTGTTCCGCATTCTGCAAGC
CTCCGGAGCGCACGTCGGCAGTCGGCTCCCTCGTTGACCGAATCACCGACCTCTCTCCCCAGGGGGATCA
TCGAATTCGCCAACATGCGGCCGCGCCACCATGAAGACGATCATCGCCCTGAGCTACATCTTCTGCCTGG
TATTCGCCGACTACAAGGACGATGATGACGCCAGCATCGATATGTATAATGGCTCCTGTTGCCGGATTGA
AGGCGACACTATCTCTCAAGTAATGCCCCCCCTCCTGATAGTGGCCTTCGTCCTGGGCGCTCTTGGTAAT
GGGGTTGCACTCTGTGGTTTCTGTTTCCACATGAAAACCTGGAAGCCCTCAACAGTGTACCTCTTTAACC
TTGCCGTGGCAGACTTCCTGCTTATGATTTGCCTGCCCTTTAGGACCGACTATTATCTGCGAcGCCGCCA
TTGGGCTTTcGGCGACATCCCCTGTCGGGtTGGTCTGTTTACTCTGGCTATGAATCGGGCCGGCAGTATC
GTCTTTCTCACTGTGGTCGCTGCCGACAGATACTTCAAGGTAGTGCACCCCCACCACGCAGTGAACACAA
TCTCCACTAGAGTTGCAGCTGGAATTGTGTGCACCCTGTGGGCTCTGGTAATCCTGGGCACAGTATACCT
GCTCCTGGAGAATCATTTGTGCGTGCAGGAGACTGCTGTGTCATGTGAATCTTTTATTATGGAGTCCGCA
AACGGGTGGCATGATATCATGTTTCAACTGGAGTTCTTTATGCCCCTTGGCATCATTCTGTTTTGCTCAT
TCAAGATCGTTTGGTCTCTCCGGCGCCGGCAGCAGCTGGCCCGGCAAGCTCGGATGAAAAAGGCCACGCG
CTTTATCATGGTTGTGGCTATCGTCTTCATCACCTGCTACCTTCCTTCCGTGTCCGCAAGACTGTATTTT
CTGTGGACCGTCCCCAGCAGCGCTTGCGATCCCAGTGTCCACGGCGCCCTCCACATCACCTTGAGCTTTA
CGTACATGAACTCTATGCTGGACCCCCTGGTGTACTATTTTAGCTCTCCCTCCTTCCCGAAATTCTATAA
TAAACTTAAGATCTGCAGCCTCAAACCAAAGCAACCAGGCCATTCCAAGACTCAGCGCCCTGAAGAGATG
CCCATTAGCAACCTTGGTAGACGGAGCTGCATCTCCGTCGCGAACTCATTTCAGTCTCAGTCCGACGGAC
AGTGGGACCCACACATTGTTGAGTGGCACATCGATACCGGTGGACGCACCCCACCCAGCCTGGGTCCCCA
AGATGAGTCCTGCACCACCGCCAGCTCCTCCCTGGCCAAGGACACTTCATCGACCGGTGAGAACCTGTAC
TTCCAGCTAAGATTAGATAAAAGTAAAGTGATTAACAGCGCATTAGAGCTGCTTAATGAGGTCGGAATCG
AAGGTTTAACAACCCGTAAACTCGCCCAGAAGCTAGGTGTAGAGCAGCCTACATTGTATTGGCATGTAAA
AAATAAGCGGGCTTTGCTCGACGCCTTAGCCATTGAGATGTTAGATAGGCACCATACTCACTTTTGCCCT
TTAGAAGGGGAAAGCTGGCAAGATTTTTTACGTAATAACGCTAAAAGTTTTAGATGTGCTTTACTAAGTC
ATCGCGATGGAGCAAAAGTACATTTAGGTACACGGCCTACAGAAAAACAGTATGAAACTCTCGAAAATCA
ATTAGCCTTTTTATGCCAACAAGGTTTTTCACTAGAGAATGCATTATATGCACTCAGCGCTGTGGGGCAT
TTTACTTTAGGTTGCGTATTGGAAGATCAAGAGCATCAAGTCGCTAAAGAAGAAAGGGAAACACCTACTA
CTGATAGTATGCCGCCATTATTACGACAAGCTATCGAATTATTTGATCACCAAGGTGCAGAGCCAGCCTT

CTTATTCGGCCTTGAATTGATCATATGCGGATTAGAAAAACAACTTAAATGTGAAAGTGGGTCCatggaa
accgtgattaaagtgattagcagcgcgccggtggtggcgatgccggtggtgattaaaaccgaaggcccgg
cgtggaccccgctggaaccggaagatacccgctggctggatggcaaacataaacgcaaaagcagccagtg
cctggtgaaaagcagcatgagcggctatattccgagctgcctggataaagatgaacagtgcgtggtgtgc
ggcgataaaccgaccggctatcattatcgctgcattacctgcgaaggctgcaaaagcttttttcgccgca
ccattcagaaaaacctgcatccgacctatagctgcacctatgatggctgctgcgtgattgataaaattac
ccgcaaccagtgccagctgtgccgctttaaaaaatgcattagcgtgggcatggcgatggatctggtgctg
gatgatagcaaacgcgtggcgaaacgcaaactgattgaagaaaaccgcgaacgccgccgcaaagaagaaa
tgattaaaagcctgcagcatcgcccgagcccgagcgcggaagaatgggaactgattcatgtggtgaccga
agcgcatcgcagcaccaacgcgcagggcagccattggaaacagcgccgcaaatttctgctggaagatatt
ggccagagcccgatggcgagcatgctggatggcgataaagtggatctggaagcgtttaccgaatttacca
aaattattaccccggcgattacccgcgtggtggattttgcgaaaaacctgccgatgtttagcgaactgcc
gtgcgaagatcagattattctgctgaaaggctgctgcatggaaattatgagcctgcgcgcggcggtgcgc
tatgatccggaaagcgaaaccctgaccctgagcggcgaaatggcggtgaaacgcgaacagctgaaaaacg
gcggcctgggcgtggtgagcgatgcgatttttgatctgggcaaaagcctgagcgcgtttaacctggatga
taccgaagtggcgctgctgcaggcggtgctgctgatgagcagcgatcgcaccggcctgatttgcgtggat
aaaattgaaaaatgccaggaaagctatctgctggcgtttgaacattatattaactatcgcaaacataaca
ttccgcatttttggagcaaactgctgatgaaagtggcggatctgcgcatgattggcgcgtatcatgcgag
ccgctttctgcatatgaaagtggaatgcccgaccgaactgagcccgcaggaagtgggcccggatcattgc
atgaaatgcgcgcattttattgatggcccgcattgcgtgaaagcgtgcccggcgggcgtgctgggcgaaa
acgataccctggtgtggaaatatgcggatgcgaacgcggtgtgccagctgtgccatccgaactgcacccg
cggctgcaaaggcccgggcctggaaggctgcccgaacggcagcaaaaccccgagcattgcggcgggcgtg
gtgggcggcctgctgtgcctggtggtggtgggcctgggcattggcctgtatctgcgccgccgccatattg
tgcgcaaacgcaccctgcgccgcctgctgcaggaacgcgaactggtggaaccgctgaccccgagcggcga
agcgccgaaccaggcgcatctgcgcattctgaaagaaaccgaatttaaaaaagtgaaagtgctgggcttt
ggcgcgtttggcaccgtgtataaaggcctgtggattccggaaggcgaaaaagtgaccattccggtggcga
ttaaagaactgcgcgaagcgaccagcccgaaagcgaacaaagaaattctggatgaagcgtatgtgatggc
gagcgtggataacccgcatgtgtgccgcctgctgggcatttgcctgaccagcaccgtgcagctgattacc
cagctgatgccgtatggctgcctgctggattatattcgcgaacataaagataacattggcagccagtatc
tgctgaactggtgcgtgcagattgcgaaaggcatgaactatctggaagaacgccatatggtgcatcgcga
tctggcggcgcgcaacgtgctggtgaaaaccccgcagcatgtgaaaattaccgattttggcctggcgaaa
cagctgggcgcggatgaaaaagaatatcatgcggaaggcggcaaagtgccgattaaatggatggcgctgg
aaagcattctgcatcgcatttatacccatcagagcgatgtgtggagctatggcgtgaccgtgtgggaact
gatgacctttggcagcaaaccgtatgatggcattccggcgagcgaaattagcagcgtgctggaaaaaggc
gaacgcctgccgcagccgccgatttgcaccattgatgtgtatatgattatggtgaaatgctggatgagcg
gcgcggatagccgcccgaaatttcgcgaactgattgcggaatttagcaaaatggcgcgcgatccgccgcg
ctatctggtgattcagggcgatgaacgcatgcatctgccgagcccgaccgatagcaaattttatcgcacc
ctgatggaagaagaagatatggaagatattgtggatgcggatgaatatctggtgccgcatcagggctttt
ttaacagcccgagcaccagccgcaccccgctgctgagcagcctgagcgcgaccagcaacaacagcgcgac
caaatgcattgatcgcaacggcggccatccggtgcgcgaagatggctttctgccggcgccggaatatgtg
aaccagctgatgccgaaaaaaccgagcaccgcgatggtgcagaaccagatttataactatattagcctga
ccgcgattagcaaactgccgatggatagccgctatcagaacagccatagcaccgcggtggataacccgga
atatctggaaCAGTGTACTAATTATGCTCTCTTGAAATTGGCTGGAGATGTCGAGTCCAACCCTGGGCCA
ATGGGTGAAAAGCCAGGTACCAGGGTCTTCAAGAAGTCGAGCCCTAACTGCAAGCTCACCGTGTACTTGG
GCAAGCGGGACTTCGTAGATCACCTGGACAAAGTGGACCCTGTAGATGGCGTGGTGCTTGTGGACCCTGA
CTACCTGAAGGACCGCAAAGTGTTTGTGACCCTCACCTGCGCCTTCCGCTATGGCCGTGAAGACCTGGAT
GTGCTGGGCTTGTCCTTCCGCAAAGACCTGTTCATCGCCACCTACCAGGCCTTCCCCCCGGTGCCCAACC
CACCCCGGCCCCCCACCCGCCTGCAGGACCGGCTGCTGAGGAAGCTGGGCCAGCATGCCCACCCCTTCTT

CTTCACCATACCCCAGAATCTTCCATGCTCCGTCACACTGCAGCCAGGCCCAGAGGATACAGGAAAGGCC
TGCGGCGTAGACTTTGAGATTCGAGCCTTCTGTGCTAAATCACTAGAAGAGAAAAGCCACAAAAGGAACT
CTGTGCGGCTGGTGATCCGAAAGGTGCAGTTCGCCCCGGAGAAACCCGGCCCCCAGCCTTCAGCCGAAAC
CACACGCCACTTCCTCATGTCTGACCGCTCCCTGCACCTCGAGGCTTCCCTGGACAAGGAGCTGTACTAC
CACGGGGAGCCCCTCAATGTAAATGTCCACGTCACCAACAACTCCACCAAGACCGTCAAGAAGATCAAAG
TCTCTGTGAGACAGTACGCCGACATCTGCCTCTTCAGCACCGCCCAGTACAAGTGTCCTGTGGCTCAACT
CGAACAAGATGACCAGGTATCTCCCAGCTCCACATTCTGTAAGGTGTACACCATAACCCCACTGCTCAGT
GACAACCGGGAGAAGCGGGGTCTCGCCCTGGATGGGAAACTCAAGCACGAGGACACCAACCTGGCTTCCA
GCACCATCGTGAAGGAGGGTGCCAACAAGGAGGTGCTGGGAATCCTGGTGTCCTACAGGGTCAAGGTGAA
GCTGGTGGTGTCTCGAGGCGGGGATGTCTCTGTGGAGCTGCCTTTTGTTCTTATGCACCCCAAGCCCCAC
GACCACATCCCCCTCCCCAGACCCCAGTCAGCCGCTCCGGAGACAGATGTCCCTGTGGACACCAACCTCA
TTGAATTTGATACCAACTATGCCACAGATGATGACATTGTGTTTGAGGACTTTGCCCGGCTTCGGCTGAA
GGGGATGAAGGATGACGACTATGATGATCAACTCTGCagcttgtttaagggaccacgtgATTACAACCCG
ATATCGAGCACCATTTGTCATTTGACGAATGAATCTGATGGGCACACAACATCGTTGTATGGTATTGGAT
TTGGTCCCTTCATCATTACAAACAAGCACTTGTTTAGAAGAAATAATGGAACACTGTTGGTCCAATCACT
ACATGGTGTATTCAAGGTCAAGAACACCACGACTTTGCAACAACACCTCATTGATGGGAGGGACATGATA
ATTATTCGCATGCCTAAGGATTTCCCACCATTTCCTCAAAAGCTGAAATTTAGAGAGCCACAAAGGGAAG
AGCGCATATGTCTTGTGACAACCAACTTCCAAACTAAGAGCATGTCTAGCATGGTGTCAGACACTAGTTG
CACATTCCCTTCATCTGATGGCATATTCTGGAAGCATTGGATTCAAACCAAGGATGGGCAGTGTGGCAGT
CCATTAGTATCAACTAGAGATGGGTTCATTGTTGGTATACACTCAGCATCGAATTTCACCAACACAAACA
ATTATTTCACAAGCGTGCCGAAAAACTTCATGGAATTGTTGACAAATCAGGAGGCGCAGCAGTGGGTTAG
TGGTTGGCGATTAAATGCTGACTCAGTATTGTGGGGGGGCCATAAAGTTTTCATGAGCAAACCTGAAGAG
CCTTTTCAGCCAGTTAAGGAAGCGACTCAACtcatgaatgaattggtgtactcgcaatgaGGATCCCCCG
GGCTGCAGGAATTCGAGCATCTTACCGCCATTTATACCCATATTTGTTCTGTTTTTCTTGATTTGGGTAT
ACATTTAAATGTTAATAAAACAAAATGGTGGGGCAATCATTTACATTTTTAGGGATATGTAATTACTAGT
TCAGGTGTATTGCCACAAGACAAACATGTTAAGAAACTTTCCCGTTATTTACGCTCTGTTCCTGTTAATC
AACCTCTGGATTACAAAATTTGTGAAAGATTGACTGATATTCTTAACTATGTTGCTCCTTTTACGCTGTG
TGGATATGCTGCTTTAATGCCTCTGTATCATGCTATTGCTTCCCGTACGGCTTTCGTTTTCTCCTCCTTG
TATAAATCCTGGTTGCTGTCTCTTTATGAGGAGTTGTGGCCCGTTGTCCGTCAACGTGGCGTGGTGTGCT
CTGTGTTTGCTGACGCAACCCCCACTGGCTGGGGCATTGCCACCACCTGTCAACTCCTTTCTGGGACTTT
CGCTTTCCCCCTCCCGATCGCCACGGCAGAACTCATCGCCGCCTGCCTTGCCCGCTGCTGGACAGGGGCT
AGGTTGCTGGGCACTGATAATTCCGTGGTGTTGTCGGGGAAGCTGACGTCCTTTCGAATTCGATATCAAG
CTTAACACGAGCCATAGATAGAATAAAAGATTTTATTTAGTCTCCAGAAAAAGGGGGGAATGAAAGACCC
CACCGCTAGCGATATCGAATTCACAACCCCTCACTCGGCGCGCCAGTCCTCCGACAGACTGAGTCGCCCG
GGTACCCGTGTTCTCAATAAACCCTCTTGCAGTTGCATCCGACTCGTGGTCTCGCTGTTCCTTGGGAGGG
TCTCCTCTGAGTGATTGACTGCCCACCTCGGGGGTCTT

The present invention also provides a kit which comprises at least two polynucleotides which between them encode a CAR, GPCR and intracellular component as described herein. In one embodiment, the kit may comprise three separate polynucleotides - each of which encodes either a CAR, GPCR or intracellular component as described herein. In one embodiment the kit may comprise two separate polynucleotides, wherein the CAR and GPCR are encoded by a first polynucleotide and the intracellular component is encoded by a second polynucleotide; or wherein the GPCR and intracellular component are encoded by a first polynucleotide and the CAR is encoded by a second polynucleotide.

### VECTOR

The present invention also provides a vector, or kit of vectors which comprises one or more polynucleotide(s) encoding a CAR, GPCR and/or intracellular component as described herein. Such a vector may be used to introduce the nucleic acid sequence(s) into a host cell so that it expresses components of a CAR signalling system according to the first aspect of the invention.

The vector may, for example, be a plasmid or a viral vector, such as a retroviral vector or a lentiviral vector, or a transposon based vector or synthetic mRNA.

The vector may be capable of transfecting or transducing a T cell or a NK cell.

### CELL

The cell may be an immune cell, such as a cytolytic immune cell. Cytolytic immune cells can be T cells or T lymphocytes which are a type of lymphocyte that play a central role in cell-mediated immunity. They can be distinguished from other lymphocytes, such as B cells and natural killer cells (NK cells), by the presence of a T-cell receptor (TCR) on the cell surface. There are various types of T cell, as summarised below.

Helper T helper cells (TH cells) assist other white blood cells in immunologic processes, including maturation of B cells into plasma cells and memory B cells, and activation of cytotoxic T cells and macrophages. TH cells express CD4 on their surface. TH cells become activated when they are presented with peptide antigens by MHC class II molecules on the surface of antigen presenting cells (APCs). These cells can differentiate into one of several subtypes, including TH1, TH2, TH3, TH17, Th9, or TFH, which secrete different cytokines to facilitate different types of immune responses.

Cytolytic T cells (TC cells, or CTLs) destroy virally infected cells and tumor cells, and are also implicated in transplant rejection. CTLs express the CD8 at their surface. These cells recognize their targets by binding to antigen associated with MHC class I, which is present on the surface of all nucleated cells. Through IL-10, adenosine and other molecules secreted by regulatory T cells, the CD8+ cells can be inactivated to an anergic state, which prevent autoimmune diseases such as experimental autoimmune encephalomyelitis.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory T cells comprise three subtypes: central memory T cells (TCM cells) and two types of effector memory T cells (TEM cells and TEMRA cells). Memory cells may be either CD4+ or CD8+. Memory T cells typically express the cell surface protein CD45RO.

Regulatory T cells (Treg cells), formerly known as suppressor T cells, are crucial for the maintenance of immunological tolerance. Their major role is to shut down T cell-mediated immunity toward the end of an immune reaction and to suppress auto-reactive T cells that escaped the process of negative selection in the thymus.

Two major classes of CD4+ Treg cells have been described - naturally occurring Treg cells and adaptive Treg cells.

Naturally occurring Treg cells (also known as CD4+CD25+FoxP3+ Treg cells) arise in the thymus and have been linked to interactions between developing T cells with both myeloid (CD11c+) and plasmacytoid (CD123+) dendritic cells that have been activated with TSLP. Naturally occurring Treg cells can be distinguished from other T cells by the presence of an intracellular molecule called FoxP3. Mutations of the FOXP3 gene can prevent regulatory T cell development, causing the fatal autoimmune disease IPEX.

Adaptive Treg cells (also known as Tr1 cells or Th3 cells) may originate during a normal immune response.

Natural Killer Cells (or NK cells) are a type of cytolytic cell which form part of the innate immune system. NK cells provide rapid responses to innate signals from virally infected cells in an MHC independent manner

NK cells (belonging to the group of innate lymphoid cells) are defined as large granular lymphocytes (LGL) and constitute the third kind of cells differentiated from the common lymphoid progenitor generating B and T lymphocytes. NK cells are known to differentiate and mature in the bone marrow, lymph node, spleen, tonsils and thymus where they then enter into the circulation.

The cell of the invention may be any of the cell types mentioned above.

T or NK cells expressing the molecules of the CAR signalling system according to the first aspect of the invention may either be created *ex vivo* either from a patient's own peripheral blood (1st party), or in the setting of a haematopoietic stem cell transplant from donor peripheral blood (2nd party), or peripheral blood from an unconnected donor (3rd party).

Alternatively, T or NK cells expressing the molecules of the CAR signalling system according to the first aspect of the invention may be derived from *ex vivo* differentiation of inducible progenitor cells or embryonic progenitor cells to T cells. Alternatively, an immortalized T-cell line which retains its lytic function and could act as a therapeutic may be used.

In all these embodiments, CAR cells are generated by introducing DNA or RNA coding for the CAR, GPCR and/or intracellular component as described herein by one of many means including transduction with a viral vector, transfection with DNA or RNA.

The CAR cell of the invention may be an *ex vivo* T or NK cell from a subject. The T or NK cell may be from a peripheral blood mononuclear cell (PBMC) sample. T or NK cells may be activated and/or expanded prior to being transduced with nucleic acid encoding the molecules providing the CAR signalling system according to the first aspect of the invention, for example by treatment with an anti-CD3 monoclonal antibody.

The cell of the invention may be made by:
(i) isolation of a T or NK cell-containing sample from a subject or other sources listed above; and
(ii) transduction or transfection of the T or NK cells with one or more polynucleotides sequence(s) as described herein encoding the CAR, GPCR and/or intracellular component as described herein.

The T or NK cells may then by purified, for example, selected on the basis of expression of the antigen-binding domain of the antigen-binding polypeptide.

Described herein is a kit which comprises a T or NK cell comprising the CAR system according to the first aspect of the invention.

### PHARMACEUTICAL COMPOSITION

The present invention also relates to a pharmaceutical composition comprising a cell, a polynucleotide or a vector according to the present invention.

In one embodiment the present invention provides a pharmaceutical composition comprising a cell according to the present invention.

In one embodiment the pharmaceutical composition comprises a plurality of immune cells expressing the CAR, GPCR and intracellular component as described herein. The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

### METHOD OF TREATMENT

Described herein is a method for treating and/or preventing a disease which comprises the step of administering the cells of the present invention (for example in a pharmaceutical composition as described above) to a subject.

A method described herein for treating a disease relates to the therapeutic use of the cells of the present invention. Herein the cells may be administered to a subject having an existing disease or condition in order to lessen, reduce or improve at least one symptom associated with the disease and/or to slow down, reduce or block the progression of the disease.

The method described herein for preventing a disease relates to the prophylactic use of the cells of the present invention. Herein such cells may be administered to a subject who has not yet contracted the disease and/or who is not showing any symptoms of the disease to prevent or impair the cause of the disease or to reduce or prevent development of at least one symptom associated with the disease. The subject may have a predisposition for, or be thought to be at risk of developing, the disease.

The method described herein may involve the steps of:
(i) isolating a T or NK cell-containing sample;
(ii) transducing or transfecting such cells with a polynucleotide or vector provided by the present invention;
(iii) administering the cells from (ii) to a subject.

The T or NK cell-containing sample may be isolated from a subject or from other sources, for example as described above. The T or NK cells may be isolated from a subject's own peripheral blood (1st party), or in the setting of a haematopoietic stem cell transplant from donor peripheral blood (2nd party), or peripheral blood from an unconnected donor (3rd party).

The methods described herein for treating a disease may involve monitoring the progression of the disease and any toxic activity and administering a small molecule drug which binds to the GCPR to the subject in order to modulate the signalling through the CAR as described herein.

Described herein, the signalling system may be a system in which binding of a ligand to the GPCR induces CAR signalling; and the method may involve administering a small molecule drug which binds to the GPCR to the subject to induce CAR signalling.

Described herein, the method may involve administering a small molecule drug which binds to the GPCR to the subject to inhibit CAR signalling and thereby reduce or lessen any adverse toxic effects.

The methods described herein for treating a disease may involve monitoring the progression of the disease and monitoring any toxic activity and adjusting the dose of the agent administered to the subject to provide acceptable levels of disease progression and toxic activity.

Monitoring the progression of the disease means to assess the symptoms associated with the disease over time to determine if they are reducing/improving or increasing/worsening.

Toxic activities relate to adverse effects caused by the CAR cells of the invention following their administration to a subject. Toxic activities may include, for example, immunological toxicity, biliary toxicity and respiratory distress syndrome.

The level of CAR expression, and therefore the level of activation of CAR cells, may be adjusted by altering the amount of small molecule drug present, or the amount of time the agent is present.

In embodiments where binding of a ligand to the GPCR induces CAR expression, the level of CAR cell activation may be augmented by increasing the dose of small molecule drug administered to the subject; or increasing the frequency of its administration. Conversely, the level of CAR cell activation may be reduced by decreasing the dose of the agent, or decreasing the frequency of administration to the subject.

In embodiments where binding of a ligand to the GPCR reduces CAR expression the level of CAR cell activation may be augmented by decreasing the dose of small molecule drug administered to the subject; or decreasing the frequency of its administration. Conversely, the level of CAR cell activation may be reduced by increasing the dose of the agent, or increasing the frequency of administration to the subject.

Higher levels of CAR cell activation are likely to be associated with reduced disease progression but increased toxic activities, whilst lower levels of CAR cell activation are likely to be associated with increased disease progression but reduced toxic activities.

Described herein is a method for treating and/or preventing a disease in a subject which subject comprises cells of the invention, which method comprises the step of administering a small molecule drug capable of binding a GPCR as described herein to the subject. As such, this method involves administering a suitable small molecule drug to a subject which already comprises CAR cells of the present invention.

As such the dose of small molecule drug administered to a subject, or the frequency of administration, may be altered in order to provide an acceptable level of both disease progression and toxic activity. The specific level of disease progression and toxic activities determined to be `acceptable' will vary according to the specific circumstances and should be assessed on such a basis. Described herein is a method for altering the activation level of the CAR cells in order to achieve this appropriate level.

The small molecule drug may be administered in the form of a pharmaceutical composition. The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

Described herein is a CAR cell, polynucleotide, vector or pharmaceutical composition of the present invention for use in treating and/or preventing a disease.

Described herein is the use of a CAR cell, polynucleotide or vector of the present invention in the manufacture of a medicament for the treatment and/or prevention of a disease.

Described herein is a small molecule drug suitable for modulating CAR expression according the present invention for use in treating and/or preventing a disease.

Described herein is a small molecule drug for use in modulating CAR expression according to the present invention in a CAR cell.

Described herein is the use of a small molecule drug suitable for modulating CAR expression according to the present invention in the manufacture of a medicament for the treatment and/or prevention of a disease.

The disease to be treated and/or prevented may be an infection, such as a viral infection.

The methods described herein may also be for the control of pathogenic immune responses, for example in autoimmune diseases, allergies and graft-vs-host rejection.

The methods described herein may be for the treatment of a cancerous disease, such as bladder cancer, breast cancer, colon cancer, endometrial cancer, kidney cancer (renal cell), leukaemia, lung cancer, melanoma, non-Hodgkin lymphoma, pancreatic cancer, prostate cancer and thyroid cancer.

The CAR cells of the present invention may be capable of killing target cells, such as cancer cells. The target cell may be recognisable by expression of a TAA, for example the expression of a TAA provided above in Table 3.

The CAR cells described herein and pharmaceutical compositions of the present invention may be for use in the treatment and/or prevention of the diseases described above.

Described herein, the CAR cells and pharmaceutical compositions of present invention may be for use in any of the methods described above.

Definitions of terms appear throughout the specification. Before the exemplary embodiments are described in more detail, it is to be understood that this disclosure is not limited to particular embodiments described, and as such may, of course, vary. It is also to be understood that the terminology used herein is for the purpose of describing particular embodiments only, and is not intended to be limiting, since the scope of the present disclosure will be limited only by the appended claims.

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

The invention will now be further described by way of Examples, which are meant to serve to assist one of ordinary skill in the art in carrying out the invention and are not intended in any way to limit the scope of the invention.

### EXAMPLES

### Example 1 - GPCR / CAR signalling system

The beta-2 adrenergic receptor (ADRB2) GPCR was used to establish a GPCR/CAR signalling system.

A retroviral vector was constructed which expresses ADRB2 fused to an artificial transcription factor via a TeV cleavage site at its carboxy-terminus. ADRB2 was modified with a FLAG epitope tag at its amino-terminus. The artificial transcription factor was generated by fusing the tetR protein with the VP16 transcription element (see Figure 4(i)).

A second retroviral vector was constructed such that eGFP was at the 5' end and whose expression was controlled by the TRE3GS promoter which contained several copies of tetO which is recognized by tetR. This retroviral vector had a second expression element under the control of the constitutively active PGK promoter. This second cassette resulted in expression of arrestin beta 2 (ARRB2) fused to the TeV protease (see Figure 4(ii)).

A third retroviral cassette was generated which was identical to the second except eGFP was replaced with a CD19-specific chimeric antigen receptor (see Figure 4(iii)). 293T cells were double transduced with either the first and the second retroviral vector, or the first and the third retroviral vectors. 293T cells were then stimulated with adrenalin (which is recognized by ADRB2).

The 293T cells were then studied by flow-cytometry for FLAG expression (to observe receptor downregulation in response to ligand). The 293T cells was also analysed for either eGFP or CAR expression. Downregulation of ADRB2 and upregulation of either eGFP or CAR was observed in response to adrenalin (see Figure 5).

A killing assay using PBMCs transduced as above with CD19+ or - targets with or without adrenaline is performed. CD19+ cells are only killed by the transduced PBMCs in the presence of adrenaline. CD19- cells are not killed by the transduced PBMCs.

### Lactate or pH-sensing GPCR/CAR signalling system

- 293T cells are transfected with HCA1 (lactate)/ GPR4 (pH) GPCR with a GFP reporter gene. Binding of lactate to HCA1 or protonation of extracellular histidines of GPR4 leads to signalling through the GPCR and expression of the GFP reporter gene.
- FACS is performed at 48hrs post-transfection, with staining for aFLAG-PE (GPCR) and GFP. Successfully transfected cells are identified as FLAG+/GFP
- Cells are treated with lactate or exposed to reduced pH as is appropriate to induce signalling through HCA1 or GPR4
- FACS is performed at 24-72 hrs post-transfection to check GFP expression
- The above experiments are repeated using a CD19-CAR instead of GFP, with FACS for CAR to identify successfully transfected cells
- Viral vectors comprising nucleic acid sequences encoding the GPCR and CAR proteins are produced and used to transduce PBMCs. The above experiments are repeated using these transduced PBMCs
- A killing assay using PBMCs transduced as above with CD19+ or - targets with or without lactate or reduced pH is performed. CD19+ cells are only killed by the transduced PBMCs in the presence of lactate or reduced pH. CD19- cells are not killed by the transduced PBMCs.

## Claims

1. A cell which co-expresses (i) a chimeric antigen receptor (CAR) and a G-protein coupled receptor (GPCR) at the cell surface and (ii) an intracellular component comprising a protease domain linked to a GPCR-targeting domain;
wherein an intracellular domain of the GPCR comprises a protease cleavage site linked to a transcriptional regulatory domain, which transcriptional regulatory domain is capable of modulating the expression of a nucleic acid encoding the CAR; and wherein the protease domain of the intracellular component is capable of cleaving at the cleavage site;
such that, upon binding of ligand to the GPCR, the intracellular component is recruited to the GPCR and the protease domain cleaves the cleavage site thereby releasing the transcriptional regulatory domain from the GPCR.

2. A cell according to claim 1 wherein
a) the transcriptional regulatory domain is an activating transcription factor domain which is capable of inducing expression of the nucleic acid encoding the CAR; optionally wherein the ligand is an entity which is increased in a tumour microenvironment compared to a non-tumour microenvironment; optionally wherein the ligand is a metabolite such as lactate, ornithine, adenosine, inosine, glutamate or kynurenic acid; or
b) the transcriptional regulatory domain is a repressor transcription factor domain which is capable of inhibiting expression of the nucleic acid encoding the CAR; optionally wherein the ligand is an entity which is reduced in a tumour microenvironment compared to a non-tumour microenvironment; optionally wherein the ligand is a metabolite such as tryptophan, glutamine or glucose.

3. A cell according to any of claims 1 or 2, wherein the GPCR is GPR81, GPR4, GPR68, GPR65, GPRC6A, GRM 1-8 or GPR35; or wherein the GPCR is GPRC6A, GRM1 or GPR1.

4. A cell according to any preceding claim wherein
a) the protease domain comprises a Tobacco Etch Virus protease, a furin protease, a tobacco vein mottling virus (TVMV) protease or a plum pox virus Nia protease; and/or
b) the GPCR-targeting domain comprises an arrestin domain; optionally wherein the arrestin domain comprises arrestin 1, arrestin beta 1, arrestin beta 2 or arrestin 3; and/or
c) the cell is a T cell.

5. A polynucleotide encoding at least two of the CAR, GPCR and intracellular component as defined in any of claims 1 to 4; optionally
a) wherein the polynucleotide encodes a CAR, GPCR and an intracellular component as defined in any of claims 1 to 4; and/or
b) wherein the polynucleotide comprises nucleic acid sequences which are co-expression sites that enable the co-expression of the CAR, GPCR and/or intracellular component; optionally wherein each co-expression site is selected from a self-cleaving peptide, a protease cleavage site and an internal ribosome entry site; and/or
c) wherein the polynucleotide comprises the following structure:
iPROM - CAR - cPROM - GPCR - CS - TF- coexpr - TarDomain/Protease
or
iREP - CAR - cPROM - GPCR - CS - Rep - coexpr - TarDomain/Protease
in which
iPROM is a transcriptional regulatory element which is capable of recruiting TF to induce expression of CAR;
iREP is a transcriptional regulatory element which is capable of recruiting Rep to inhibit expression of CAR;
CAR is a nucleic acid sequence encoding a chimeric antigen receptor;
cPROM is a constitutively active promoter which drives expression of the GPCR and TarDomain/Protease;
GPCR is a nucleic acid sequence encoding a G-protein coupled receptor;
CS is a nucleic acid sequence which comprises a cleavage site for a protease; TF is a nucleic acid sequence encoding an activating transcription factor domain which is capable of promoting the expression of the nucleic acid encoding the CAR; Rep is a nucleic acid sequence encoding a repressor domain which is capable of inhibiting the expression of the nucleic acid encoding the CAR; TarDomain/Protease is a nucleic acid sequence encoding a GPCR-targeting domain linked to a protease domain; and
coexpr is a nucleic acid sequence enabling co-expression of the activating transcription factor domain or repressor domain and the TarDomain/Protease; optionally wherein coexpr encodes an amino acid sequence comprising a self-cleaving peptide.

6. A kit which comprises at least two polynucleotides which between them encode a CAR, GPCR and intracellular component as defined in any of claims 1 to 4; optionally wherein
a) the CAR, GPCR and intracellular component are each encoded by a separate polynucleotide; or
b) the kit comprises
(i) a polynucleotide which comprises the following structure:
cPROM - GPCR - CS - TF- coexpr - TarDomain/Protease
and
(ii) a polynucleotide which comprises the following structure:
iPROM - CAR
or
(i) a polynucleotide which comprises the following structure:
cPROM - GPCR - CS - Rep - coexpr - TarDomain/Protease
and
(ii) a polynucleotide which comprises the following structure:
iREP - CAR
in which
cPROM is a constitutively active promoter which drives expression of the GPCR and TarDomain/Protease
GPCR is a nucleic acid sequence encoding a G-protein coupled receptor CS is a nucleic acid sequence which comprises a cleavage site for the protease TF is a nucleic acid sequence encoding an activating transcription factor domain which is capable of promoting the expression of the nucleic acid encoding the CAR Rep is a nucleic acid sequence encoding a repressor domain which is capable of inhibiting the expression of the nucleic acid encoding the CAR TarDomain/Protease is a nucleic acid sequence encoding a GPCR-targeting domain and a protease
coexpr is a nucleic acid sequence enabling co-expression of the transcription factor domain or repressor domain and the TarDomain/Protease;
iPROM is a transcriptional regulatory element which is capable of recruiting TF to induce expression of CAR
iREP is a transcriptional regulatory element which is capable of recruiting Rep to inhibit expression of CAR; and
CAR is a nucleic acid sequence encoding a chimeric antigen receptor.

7. A vector comprising a polynucleotide according to claim 5; optionally wherein the vector is an integrating viral vector or a transposon.

8. A kit comprising a plurality of vectors each comprising a polynucleotide as defined in claim 5; optionally wherein the vectors are integrating viral vectors or transposons.

9. A kit which comprises a polynucleotide or a plurality of polynucleotides as defined in claims 5 or 6 or a vector or a plurality of vectors as defined in claims 7 or 8 and a small molecule drug which is capable of binding to the GPCR.

10. A method for making a cell according to any of claims 1 to 4, which comprises the step of introducing a polynucleotide or a plurality of polynucleotides as defined in claims 5 or 6 or a vector or a plurality of vectors as defined in claims 7 or 8 into the cell; optionally wherein the cell is from a sample isolated from a subject.

11. A pharmaceutical composition which comprises a cell according to any of claims 1 to 4, a polynucleotide according to claim 5 or a vector or plurality of vectors according to claims 7 or 8.

12. A pharmaceutical composition as defined in claim 11 for use in treating and/or preventing a disease in a subject.

13. A pharmaceutical composition for use according to claim 12 wherein
a) the pharmaceutical composition comprises:
cells that have been isolated from a subject and transduced or transfected with a polynucleotide or a plurality of polynucleotides as defined in claims 5 or 6 or a vector or a plurality of vectors as defined in claims 7 or 8; and/or
b) the pharmaceutical composition is administered in combination with a small molecule drug which binds to the GPCR in order to induce expression of a CAR as defined in any of claims 1 to 4; optionally wherein
the progression of disease and/or toxic activity in the subject is monitored and the dose of small molecule drug is adjusted to provide acceptable levels of disease progression and/or toxic activity; and/or
c) the pharmaceutical composition is administered in combination with a small molecule drug which binds to the GPCR in order to inhibit expression of a CAR as defined in any of claims 1 to 4; optionally wherein toxic activity in the subject is monitored and a small molecule drug is administered which is capable of binding to the GPCR to the subject to reduce adverse toxic effects.

14. A pharmaceutical composition for use according to claim 13, which involves monitoring the progression of disease and/or monitoring toxic activity in the subject and comprises the step of administering a small molecule drug which is capable of binding to the GPCR to the subject to provide acceptable levels of disease progression and/or toxic activity.

15. The pharmaceutical composition for use according to any of claims 12 to 14 wherein the disease is a cancer.

## Patentansprüche

1. Zelle, die (i) einen chimären Antigenrezeptor (CAR) und einen G-Protein-gekoppelten Rezeptor (GPCR) an der Zelloberfläche und (ii) eine intrazelluläre Komponente, die eine mit einer GPCR-Targeting-Domäne verknüpfte Proteasedomäne umfasst, coexprimiert;
wobei eine intrazelluläre Domäne des GPCR eine mit einer Transkriptionsregulatordomäne verknüpfte Protease-Spaltstelle umfasst, wobei die Transkriptionsregulatordomäne die Expression einer den CAR codierenden Nukleinsäure modulieren kann; und wobei die Proteasedomäne der intrazellulären Komponente zur Spaltung an der Spaltstelle fähig ist;
so dass nach Ligandenbindung an den GPCR die intrazelluläre Komponente zum GPCR rekrutiert wird und die Proteasedomäne die Spaltstelle spaltet, wodurch die Transkriptionsregulatordomäne vom GPCR gelöst wird.

2. Zelle nach Anspruch 1, wobei
a) es sich bei der Transkriptionsregulatordomäne um eine aktivierende Transkriptionsfaktordomäne handelt, die die Expression der den CAR codierenden Nukleinsäure induzieren kann; gegebenenfalls wobei es sich bei dem Liganden um ein Gebilde handelt, das in einem Tumormikromilieu im Vergleich zu einem Nichttumormikromilieu vermehrt vorliegt; gegebenenfalls wobei es sich bei dem Liganden um einen Metaboliten wie Lactat, Ornithin, Adenosin, Inosin, Glutamat oder Kynurensäure handelt; oder
b) es sich bei der Transkriptionsregulatordomäne um eine Repressor-Transkriptionsfaktordomäne handelt, die die Expression der den CAR codierenden Nukleinsäure hemmen kann; gegebenenfalls wobei es sich bei dem Liganden um ein Gebilde handelt, das in einem Tumormikromilieu im Vergleich zu einem Nichttumormikromilieu vermindert vorliegt; gegebenenfalls wobei es sich bei dem Liganden um einen Metaboliten wie Tryptophan, Glutamin oder Glucose handelt.

3. Zelle nach Anspruch 1 oder Anspruch 2, wobei es sich bei dem GPCR um GPR81, GPR4, GPR68, GPR65, GPRC6A, GRM 1-8 oder GPR35 handelt; oder wobei es sich bei dem GPCR um GPRC6A, GRM1 oder GPR1 handelt.

4. Zelle nach einem vorhergehenden Anspruch, wobei
a) die Proteasedomäne eine Tobacco Etch Virus-Protease, eine Furin-Protease, eine TVMV(Tobacco Vein Mottling Virus)-Protease oder eine Pflaumen-Scharka-Virus-Nia-Proteaseumfasst; und/oder
b) die GPCR-Targeting-Domäne eine Arrestindomäne umfasst; gegebenenfalls wobei die Arrestindomäne Arrestin 1, Arrestin-beta 1, Arrestin-beta 2 oder Arrestin 3 umfasst; und/oder
c) es sich bei der Zelle um eine T-Zelle handelt.

5. Polynukleotid, codierend wenigstens zwei von dem CAR, dem GPCR und der intrazellulären Domäne gemäß einem der Ansprüche 1 bis 4; gegebenenfalls
a) wobei das Polynukleotid einen CAR, GPCR und eine intrazelluläre Komponente gemäß einem der Ansprüche 1 bis 4 codiert; und/oder
b) wobei das Polynukleotid Nukleinsäuresequenzen umfasst, bei denen es sich um Coexpressionsstellen handelt, die die Coexpression des CAR, des GPCR und/oder der intrazellulären Komponente ermöglichen; gegebenenfalls wobei die Coexpressionsstellen jeweils aus einem selbstspaltenden Peptid, einer Protease-Spaltstelle und einer internen Ribosomeeintrittstelle ausgewählt sind; und/oder
c) wobei das Polynukleotid die folgende Struktur umfasst:
iPROM - CAR - cPROM - GPCR - CS -TF-coexpr - TarDomain/Protease
oder
iREP - CAR - cPROM - GPCR - CS - Rep -coexpr - TarDomain/Protease
worin
iPROM für ein Transkriptionsregulatorelement steht, das TF zur Induktion der Expression von CAR rekrutieren kann; iREP für ein Transkriptionsregulatorelement steht, das Rep zur Hemmung der Expression von CAR rekrutieren kann; CAR für eine Nukleinsäuresequenz steht, die einen chimären Antigenrezeptor codiert;
cPROM für einen konstitutiv aktiven Promotor steht, der die Expression des GPCR und der TarDomain/Protease treibt;
GPCR für eine Nukleinsäuresequenz steht, die einen G-Protein-gekoppelten Rezeptor codiert;
CS für eine Nukleinsäuresequenz steht, die eine Spaltstelle für eine Protease umfasst;
TF für eine Nukleinsäuresequenz steht, die eine aktivierende Transkriptionsfaktordomäne codiert, die die Expression der den CAR codierenden Nukleinsäure fördern kann;
Rep für eine Nukleinsäuresequenz steht, die eine Repressor-Transkriptionsfaktordomäne codiert, die die Expression der den CAR codierenden Nukleinsäure hemmen kann;
TarDomain/Protease für eine Nukleinsäuresequenz steht, die eine mit einer Proteasedomäne verknüpfte GPCR-Targeting-Domäne codiert; und
coexpr für eine Nukleinsäuresequenz steht, die die Coexpression der aktivierenden Transkriptionsfaktordomäne oder Repressordomäne und der TarDomain/Protease ermöglicht;
gegebenenfalls wobei coexpr eine Aminosäuresequenz codiert, die ein selbstspaltendes Peptid umfasst.

6. Kit, das wenigstens zwei Polynukleotide umfasst, die zusammengenommen einen CAR, einen GPCR und eine intrazelluläre Komponente gemäß einem der Ansprüche 1 bis 4 codieren; gegebenenfalls wobei
a) der CAR, der GPCR bzw. die intrazelluläre Komponente jeweils durch ein eigenes Polynukleotid codiert sind; oder
b) das Kit
(i) ein Polynukleotid, das die folgende Struktur umfasst:
cPROM - GPCR - CS - TF- coexpr - TarDomain/Protease
und
(ii) ein Polynukleotid, das die folgende Struktur umfasst:
iPROM-CAR
oder
(i) ein Polynukleotid, das die folgende Struktur umfasst:
cPROM - GPCR - CS - Rep - coexpr - TarDomain/Protease
und
(ii) ein Polynukleotid, das die folgende Struktur umfasst:
iREP-CAR
umfasst, worin
cPROM für einen konstitutiv aktiven Promotor steht, der die Expression des GPCR und der TarDomain/Protease treibt,
GPCR für eine Nukleinsäuresequenz steht, die einen G-Protein-gekoppelten Rezeptor codiert,
CS für eine Nukleinsäuresequenz steht, die eine Spaltstelle für die Protease umfasst,
TF für eine Nukleinsäuresequenz steht, die eine aktivierende Transkriptionsfaktordomäne codiert, die die Expression der den CAR codierenden Nukleinsäure fördern kann,
Rep für eine Nukleinsäuresequenz steht, die eine Repressor-Transkriptionsfaktordomäne codiert, die die Expression der den CAR codierenden Nukleinsäure hemmen kann,
TarDomain/Protease für eine Nukleinsäuresequenz steht, die eine GPCR-Targeting-Domäne und eine Protease codiert, coexpr für eine Nukleinsäuresequenz steht, die die Coexpression der Transkriptionsfaktordomäne oder Repressordomäne und der TarDomain/Protease ermöglicht;
iPROM für ein Transkriptionsregulatorelement steht, das TF zur Induktion der Expression von CAR rekrutieren kann, iREP für ein Transkriptionsregulatorelement steht, das Rep zur Hemmung der Expression von CAR rekrutieren kann; und
CAR für eine Nukleinsäuresequenz steht, die einen chimären Antigenrezeptor codiert.

7. Vektor, umfassend ein Polynukleotid nach Anspruch 5; gegebenenfalls wobei es sich bei dem Vektor um einen integrierenden Virusvektor oder ein Transposon handelt.

8. Kit, umfassend mehrere Vektoren, die jeweils ein Polynukleotid gemäß Anspruch 5 umfassen; gegebenenfalls wobei es sich bei den Vektoren um integrierende Virusvektoren oder Transposons handelt.

9. Kit, das ein Polynukleotid oder mehrere Polynukleotide gemäß Anspruch 5 oder 6 oder einen Vektor oder mehrere Vektoren gemäß Anspruch 7 oder 8 sowie einen niedermolekularen Arzneistoff, der an den GPCR binden kann, umfasst.

10. Verfahren zur Erzeugung einer Zelle nach einem der Ansprüche 1 bis 4, das den Schritt umfasst, bei dem ein Polynukleotid oder mehrere Polynukleotide gemäß Anspruch 5 oder 6 oder ein Vektor oder mehrere Vektoren gemäß Anspruch 7 oder 8 in die Zelle eingeführt wird bzw. werden; gegebenenfalls wobei die Zelle aus einer von einem Individuum isolierten Probe stammt.

11. Pharmazeutische Zusammensetzung, die eine Zelle nach einem der Ansprüche 1 bis 4, ein Polynukleotid nach Anspruch 5 oder einen Vektor oder mehrere Vektoren nach Anspruch 7 oder 8 umfasst.

12. Pharmazeutische Zusammensetzung gemäß Anspruch 11 zur Verwendung bei der Behandlung und/oder Vorbeugung einer Krankheit bei einem Individuum.

13. Pharmazeutische Zusammensetzung zur Verwendung nach Anspruch 12, wobei
a) die pharmazeutische Zusammensetzung Folgendes umfasst:
Zellen, die von einem Individuum isoliert und mit einem Polynukleotid oder mehreren Polynukleotiden gemäß Anspruch 5 oder 6 oder einem Vektor oder mehreren Vektoren gemäß Anspruch 7 oder 8 transduziert oder transfiziert wurden; und/oder
b) die pharmazeutische Zusammensetzung in Kombination mit einem niedermolekularen Arzneistoff, der an den GPCR bindet, verabreicht wird, um die Expression eines CAR gemäß einem der Ansprüche 1 bis 4 zu induzieren; gegebenenfalls wobei
die Krankheitsprogression und/oder toxische Aktivität beim Individuum überwacht werden/wird und die Dosis an niedermolekularem Arzneistoff so eingestellt wird, dass akzeptierbare Niveaus von Krankheitsprogression und/oder toxischer Aktivität bereitgestellt werden; und/oder
c) die pharmazeutische Zusammensetzung in Kombination mit einem niedermolekularen Arzneistoff, der an den GPCR bindet, verabreicht wird, um die Expression eines CAR gemäß einem der Ansprüche 1 bis 4 zu hemmen; gegebenenfalls wobei toxische Aktivität beim Individuum überwacht und dem Individuum ein niedermolekularer Arzneistoff, der an den GPCR binden kann, verabreicht wird, um toxische Nebenwirkungen zu verringern.

14. Pharmazeutische Zusammensetzung zur Verwendung nach Anspruch 13, was Überwachen der Krankheitsprogression und/oder Überwachen von toxischer Aktivität beim Individuum beinhaltet und den Schritt umfasst, bei dem dem Individuum ein niedermolekularer Arzneistoff, der an den GPCR binden kann, verabreicht wird, um akzeptierbare Niveaus von Krankheitsprogression und/oder toxischer Aktivität bereitzustellen.

15. Pharmazeutische Zusammensetzung zur Verwendung nach einem der Ansprüche 12 bis 14, wobei es sich bei der Krankheit um eine Krebserkrankung handelt.

## Revendications

1. Cellule qui co-exprime (i) un récepteur antigénique chimérique (CAR) et un récepteur couplé à une protéine G (GPCR) à la surface de la cellule et (ii) un composant intracellulaire comprenant un domaine de protéase lié à un domaine ciblant le GPCR ;
dans laquelle un domaine intracellulaire du GPCR comprend un site de clivage de protéase lié à un domaine de régulation transcriptionnelle, lequel domaine de régulation transcriptionnelle est capable de moduler l'expression d'un acide nucléique codant pour le CAR ; et dans laquelle le domaine de protéase du composant intracellulaire est capable de se cliver au niveau du site de clivage ;
de sorte que, lorsqu'un ligand se lie au GPCR, le composant intracellulaire est recruté pour le GPCR et le domaine de protéase clive le site de clivage, libérant ainsi le domaine de régulation transcriptionnelle du GPCR.

2. Cellule selon la revendication 1 dans laquelle
a) le domaine de régulation transcriptionnelle est un domaine de facteur de transcription activateur qui est capable d'induire l'expression de l'acide nucléique codant pour le CAR ; éventuellement le ligand étant une entité qui est augmentée dans un microenvironnement tumoral par rapport à un microenvironnement non tumoral ; éventuellement le ligand étant un métabolite tel que le lactate, l'ornithine, l'adénosine, l'inosine, le glutamate ou l'acide kynurénique ; ou
b) le domaine de régulation transcriptionnelle est un domaine de facteur de transcription répresseur qui est capable d'inhiber l'expression de l'acide nucléique codant pour le CAR ; éventuellement le ligand étant une entité qui est réduite dans un microenvironnement tumoral par rapport à un microenvironnement non tumoral ;
éventuellement le ligand étant un métabolite tel que le tryptophane, la glutamine ou le glucose.

3. Cellule selon l'une quelconque des revendications 1 ou 2, dans laquelle le GPCR est GPR81, GPR4, GPR68, GPR65, GPRC6A, GRM 1-8 ou GPR35 ; ou dans laquelle le GPCR est GPRC6A, GRM1 ou GPR1.

4. Cellule selon une quelconque revendication précédente,
a) le domaine de protéase comprenant une protéase du virus Tobacco Etch, une protéase de la furine, une protéase du virus de la marbrure des veines du tabac (TVMV) ou une protéase Nia du virus de la variole du prunier ; et/ou
b) le domaine ciblant le GPCR comprenant un domaine d'arrestine ; éventuellement le domaine d'arrestine comprenant l'arrestine 1, l'arrestine bêta 1, l'arrestine bêta 2 ou l'arrestine 3 ; et/ou
c) la cellule étant une cellule T.

5. Polynucléotide codant pour au moins deux parmi le CAR, le GPCR et le composant intracellulaire tels que définis dans l'une quelconque des revendications 1 à 4 ; éventuellement
a) le polynucléotide codant pour un CAR, un GPCR et un composant intracellulaire tels que définis dans l'une quelconque des revendications 1 à 4 ; et/ou
b) le polynucléotide comprenant des séquences d'acides nucléiques qui sont des sites de co-expression permettant la co-expression du CAR, du GPCR et/ou du composant intracellulaire ; éventuellement dans lequel chaque site de co-expression est choisi parmi un peptide auto-clivant, un site de clivage de protéase et un site d'entrée de ribosome interne ; et/ou
c) le polynucléotide comprenant la structure suivante
iPROM-CAR-cPROM-GPCR-CS-TF-coexpr-TarDomaine/Protéase ou iREP-CAR-cPROM-GPCR-CS-Rep-coexpr-TarDomaine/Protéase dans laquelle
iPROM est un élément de régulation transcriptionnelle capable de recruter TF pour induire l'expression de CAR ;
iREP est un élément de régulation transcriptionnelle capable de recruter Rep pour inhiber l'expression de CAR ;
CAR est une séquence d'acides nucléiques codant pour un récepteur antigénique chimérique ;
cPROM est un promoteur constitutivement actif qui entraîne l'expression du GPCR et de la TarDomaine/Protéase ;
GPCR est une séquence d'acides nucléiques codant pour un récepteur couplé à une protéine G ;
CS est une séquence d'acides nucléiques qui comprend un site de clivage pour une protéase ;
TF est une séquence d'acides nucléiques codant pour un domaine de facteur de transcription activateur capable de promouvoir l'expression de l'acide nucléique codant pour le CAR ;
Rep est une séquence d'acides nucléiques codant pour un domaine répresseur capable d'inhiber l'expression de l'acide nucléique codant pour le CAR ; TarDomaine/Protéase est une séquence d'acides nucléiques codant pour un domaine ciblant le GPCR lié à un domaine de protéase ; et
coexpr est une séquence d'acides nucléiques permettant la co-expression du domaine de facteur de transcription activateur ou du domaine répresseur et de la TarDomaine/Protéase ;
éventuellement dans lequel coexpr code pour une séquence d'acides aminés comprenant un peptide auto-clivant.

6. Kit qui comprend au moins deux polynucléotides qui entre eux codent pour un CAR, un GPCR et un composant intracellulaire tels que définis dans l'une quelconque des revendications 1 à 4 ; éventuellement
a) le CAR, le GPCR et le composant intracellulaire étant chacun codés par un polynucléotide distinct ; ou
b) le kit comprenant
(i) un polynucléotide qui comprend la structure suivante :
cPROM-GPCR-CS-TF-coexpr-TarDomaine/Protéase
et
(ii) un polynucléotide qui comprend la structure suivante :
iPROM-CAR
ou
(i) un polynucléotide qui comprend la structure suivante :
cPROM-GPCR-CS-Rep-coexpr-TarDomaine/Protéase
et
(ii) un polynucléotide qui comprend la structure suivante :
iREP-CAR
dans lesquels
cPROM est un promoteur constitutivement actif qui entraîne l'expression du GPCR et de la TarDomaine/Protéase
GPCR est une séquence d'acides nucléiques codant pour un récepteur couplé à une protéine G
CS est une séquence d'acides nucléiques qui comprend un site de clivage pour la protéase
TF est une séquence d'acides nucléiques codant pour un domaine de facteur de transcription activateur capable de promouvoir l'expression de l'acide nucléique codant pour le CAR
Rep est une séquence d'acides nucléiques codant pour un domaine répresseur capable d'inhiber l'expression de l'acide nucléique codant pour le CAR
TarDomaine/Protéase est une séquence d'acides nucléiques codant pour un domaine ciblant le GPCR et une protéase coexpr est une séquence d'acides nucléiques permettant la co-expression du domaine de facteur de transcription ou du domaine répresseur et de la TarDomaine/Protéase ;
iPROM est un élément de régulation transcriptionnelle capable de recruter TF pour induire l'expression de CAR iREP est un élément de régulation transcriptionnelle capable de recruter Rep pour inhiber l'expression de CAR ; et
CAR est une séquence d'acides nucléiques codant pour un récepteur antigénique chimérique.

7. Vecteur comprenant un polynucléotide selon la revendication 5 ; éventuellement le vecteur étant un vecteur viral intégrateur ou un transposon.

8. Kit comprenant une pluralité de vecteurs comprenant chacun un polynucléotide tel que défini dans la revendication 5 ; éventuellement dans lequel les vecteurs sont des vecteurs viraux intégrateurs ou des transposons.

9. Kit qui comprend un polynucléotide ou une pluralité de polynucléotides tel(s) que défini(s) dans les revendications 5 ou 6 ou un vecteur ou une pluralité de vecteurs tel(s) que défini(s) dans les revendications 7 ou 8 et un médicament à petite molécule capable de se lier au GPCR.

10. Procédé de fabrication d'une cellule selon l'une quelconque des revendications 1 à 4, qui comprend l'étape d'introduction d'un polynucléotide ou d'une pluralité de polynucléotides tel(s) que défini(s) dans les revendications 5 ou 6, ou d'un vecteur ou d'une pluralité de vecteurs tel(s) que défini(s) dans les revendications 7 ou 8 dans la cellule ; éventuellement la cellule provenant d'un échantillon isolé d'un sujet.

11. Composition pharmaceutique qui comprend une cellule selon l'une quelconque des revendications 1 à 4, un polynucléotide selon la revendication 5 ou un vecteur ou une pluralité de vecteurs selon les revendications 7 ou 8.

12. Composition pharmaceutique telle que définie dans la revendication 11 pour une utilisation dans le traitement et/ou la prévention d'une maladie chez un sujet.

13. Composition pharmaceutique pour une utilisation selon la revendication 12,
a) la composition pharmaceutique comprenant :
des cellules qui ont été isolées d'un sujet et transduites ou transfectées avec un polynucléotide ou une pluralité de polynucléotides tel(s) que défini(s) dans les revendications 5 ou 6 ou un vecteur ou une pluralité de vecteurs tel(s) que défini(s) dans les revendications 7 ou 8 ; et/ou
b) la composition pharmaceutique étant administrée en combinaison avec un médicament à petite molécule qui se lie au GPCR afin d'induire l'expression d'un CAR tel que défini dans l'une quelconque des revendications 1 à 4 ; éventuellement
la progression de la maladie et/ou l'activité toxique chez le sujet étant surveillée(s) et la dose de médicament à petite molécule étant ajustée pour fournir des niveaux acceptables de progression de la maladie et/ou d'activité toxique ; et/ou
c) la composition pharmaceutique étant administrée en association avec un médicament à petite molécule qui se lie au GPCR afin d'inhiber l'expression d'un CAR tel que défini dans l'une quelconque des revendications 1 à 4 ; éventuellement l'activité toxique chez le sujet étant surveillée et un médicament à petite molécule étant administré, lequel est capable de se lier au GPCR, au sujet afin de réduire des effets toxiques indésirables.

14. Composition pharmaceutique pour une utilisation selon la revendication 13, qui implique le suivi de la progression de la maladie et/ou le suivi de l'activité toxique chez le sujet et comprend l'étape d'administration d'un médicament à petite molécule capable de se lier au GPCR au sujet afin de fournir des niveaux acceptables de progression de la maladie et/ou d'activité toxique.

15. Composition pharmaceutique pour une utilisation selon l'une quelconque des revendications 12 à 14, dans laquelle la maladie est un cancer.
